# EUROPEAN PATENT SPECIFICATION

(11) **EP 1 783 214 B1**
(45) Date of publication and mention of the grant of the patent: **27.06.2012**
(21) Application number: 05766163.9
(22) Date of filing: 21.07.2005
(51) Int. Cl.: C12N 9/10, C07K 14/00, C12N 15/09, C12P 21/00, C12P 21/02

(54) **MUTANT GLYCOPROTEIN RESISTANT TO MODIFICATION WITH ASPARAGINE-LINKED SUGAR CHAIN**
MUTANTES, NICHT MIT EINER ASPARAGIN-VERKNÜPFTEN ZUCKERKETTE MODIFIZIERTES GLYCOPROTEIN
GLYCOPROTEINE MUTANTE NON MODIFIEE PAR UNE CHAÎNE DE SUCRE LIEE A UN RESIDU D'ASPARAGINE

(30) Priority: 21.07.2004 JP 2004213616
(43) Date of publication of application: 09.05.2007
(73) Proprietor: SEIKAGAKU CORPORATION, Chiyoda-ku, Tokyo 100-0005 (JP)
(72) Inventor: INOKUCHI, Jin-ichi, Sendai-shi, Miyagi 981-3203 (JP); UEMURA, Satoshi, Sendai-shi, Miyagi 981-0905 Japan (JP)
(74) Representative: Elsy, David
(86) International application number: PCT/JP2005/013424
(87) International publication number: WO 2006/009226

(56) References cited:
- WO-A-93/18157
- WO-A1-2005/058951
- JP-A- 09 501 317
- VANCE B A ET AL: "Distinct but Dispensable N-Glycosylation of Human CD69 Proteins" ARCHIVES OF BIOCHEMISTRY AND BIOPHYSICS, NEW YORK, US, US, vol. 368, no. 2, 15 August 1999 (1999-08-15), pages 214-220, XP002998874 ISSN: 0003-9861
- DATABASE UniProt [Online] 5 July 2004 (2004-07-05), "Ganglioside GM3 synthase (EC <A HREF="http://srs.ebi.ac.uk/srsbin/cgi-bin/ wgetz?[enzyme-ECNumber:2.4.99.9]+-e">2.4.9 9.9</A>) (Alpha-2,3-sialyltransferase). VSLAGFGYNL SQKEAPLHYY DSVPMTTILK EAMHNVQKET VFLKRLVASG SITDLTGGIH" XP002467899 retrieved from EBI accession no. UNIPROT:Q705K5 Database accession no. Q705K5
- JEANNEAU CHARLOTTE ET AL: "Structure-function analysis of the human sialyltransferase ST3Gal I - Role of N-glycosylation and a novel conserved sialylmotif" JOURNAL OF BIOLOGICAL CHEMISTRY, vol. 279, no. 14, 2 April 2004 (2004-04-02), pages 13461-13468, XP002467895 ISSN: 0021-9258
- CHEN CHUN ET AL: "Minimal structural and glycosylation requirements for ST6Gal I activity and trafficking" GLYCOBIOLOGY, vol. 10, no. 5, May 2000 (2000-05), pages 531-583, XP002467896 ISSN: 0959-6658
- CLOSE B E ET AL: "Polysialyltransferase-1 autopolysialylation is not requisite for polysialylation of neural cell adhesion molecule" JOURNAL OF BIOLOGICAL CHEMISTRY, AMERICAN SOCIETY OF BIOLOCHEMICAL BIOLOGISTS, BIRMINGHAM,, US, vol. 275, no. 6, 11 February 2000 (2000-02-11), pages 4484-4491, XP003016260 ISSN: 0021-9258
- CLOSE B E ET AL: "The polysialyltransferase ST8Sia II/STX: posttranslational processing and role of autopolysialylation in the polysialylation of neural cell adhesion molecule" GLYCOBIOLOGY, IRL PRESS,, GB, vol. 11, no. 11, November 2001 (2001-11), pages 997-1008, XP003016259 ISSN: 0959-6658
- UEMURA SATOSHI ET AL: "Substitution of the N-glycan function in glycosyltransferases by specific amino acids: ST3Gal-V as a model enzyme" GLYCOBIOLOGY, vol. 16, no. 3, March 2006 (2006-03), pages 258-270, XP002467897 ISSN: 0959-6658
- DATABASE BIOSIS [Online] BIOSCIENCES INFORMATION SERVICE, PHILADELPHIA, PA, US; November 2003 (2003-11), UEMURA SATOSHI ET AL: "Role of N-linked glycans of ganglioside GM3 synthase (SAT-I)." XP002467900 Database accession no. PREV200300585066 -& GLYCOBIOLOGY, vol. 13, no. 11, November 2003 (2003-11), pages 855-856, XP002467898 8TH ANNUAL CONFERENCE OF THE SOCIETY FOR GLYCOBIOLOGY; SAN DIEGO, CALIFORNIA, USA; DECEMBER 03-06, 2003 ISSN: 0959-6658
- M]HLENHOFF M ET AL: "The Impact of N-Glycosylation on the Functions of Polysialyltransferases" JOURNAL OF BIOLOGICAL CHEMISTRY, AMERICAN SOCIETY OF BIOLOCHEMICAL BIOLOGISTS, BIRMINGHAM,, US, vol. 276, no. 36, 7 September 2001 (2001-09-07), pages 34066-34073, XP002998875 ISSN: 0021-9258
- VANCE B A ET AL: 'Distinct but Dispensable N-Glycosylation of Human CD69 Proteins.' ARCHIVES OF BIOCHEMISTRY AND BIOPHYSICS. vol. 368, no. 2, 1999, pages 214 - 220, XP002998874
- MUHLENHOFF M ET AL: 'The Impact of N-Glycosylation on the Functions of Polysialyltransferases.' J BIOL CHEM. vol. 276, no. 36, 2001, pages 34066 - 34073, XP002998875
- OKA S ET AL: 'Idenshi Sosa o Mochiita Tosa no Sakujo Oyobi Kaihen.' PROTEIN, NUCLEIC ACID AND ENZYME. vol. 37, no. 3, 1992, pages 389 - 394, XP002998876
- KONO MARI ET AL: "Molecular cloning and functional expression of a fifth-type alpha2,3-sialyltransferase (mST3Gal V: GM 3 synthase)", BIOCHEMICAL AND BIOPHYSICAL RESEARCH COMMUNICATIONS, vol. 253, no. 1, 9 December 1998 (1998-12-09), pages 170-175, ISSN: 0006-291X

### SEQUENCE LISTING

<110> Seikagaku Corporation
   Jin-ichi Inokuchi
<120> MUTANT GLYCOPROTEIN RESISTANT TO MODIFICATION WITH ASPARAGINE-LINKED SUGAR CHAIN
<130> F200505C5144
<150> JP 2004-213616
   <151> 2004-07-21
<160> 43
<170> PatentIn version 3.1
<210> 1
   <211> 3
   <212> PRT
   <213> Artificial
<220>
   <223> A partial amino acid sequence of glycoprotein wherein Xaa at position 2 =an amino acid other than Pro, Xaa at position 3 = Thr or Ser.
<400> 1
   Asn Xaa Xaa
   1
<210> 2
   <211> 6
   <212> PRT
   <213> Artificial
<220>
   <223> A partial amino acid sequence of glycoprotein wherein Xaa at position 1 = His or Asp, Xaa at position 5 = an amino acid other than Pro, Xaa at position 6 = Thr or Ser.
<400> 2
   Xaa Val Gly Asn Xaa Xaa
   1 5
<210> 3
   <211> 1080
   <212> DNA
   <213> Mus musculus
<220>
   <221> CDS
   <222> (1) .. (1080)
   <223>
<400> 3
   atg aga aga ccc agc ttg tta ata aaa gac atc tgc aag tgc acg ttg      48
   Met Arg Arg Pro Ser Leu Leu Ile Lys Asp Ile Cys Lys Cys Thr Leu
   1               5                   10                  15
   gtt gca ttt gga gtc tgg ctc ctg tac atc ctc att ttg aat tac acc      96
   Val Ala Phe Gly Val Trp Leu Leu Tyr Ile Leu Ile Leu Asn Tyr Thr
               20                  25                  30
   gct gaa gaa tgt gac atg aaa aga atg cac tat gtg gac cct gac cgg      144
   Ala Glu Glu Cys Asp Met Lys Arg Met His Tyr Val Asp Pro Asp Arg
           35                  40                  45
   ata aag aga gct cag agc tat gct cag gaa gtc ttg cag aag gaa tgt      192
   Ile Lys Arg Ala Gln Ser Tyr Ala Gln Glu Val Leu Gln Lys Glu Cys
       50                  55                  60
   cgg ccc agg tac gcg aag acg gct atg gct ctg tta ttt gag gac agg      240
   Arg Pro Arg Tyr Ala Lys Thr Ala Met Ala Leu Leu Phe Glu Asp Arg
   65                  70                  75                  80
   tac agc atc aac ttg gag cct ttt gtg cag aag gtc ccc acg gcc agt      288
   Tyr Ser Ile Asn Leu Glu Pro Phe Val Gln Lys Val Pro Thr Ala Ser
                   85                  90                  95
   gaa gct gag ctc aag tat gac ccg cct ttt gga ttc cgg aag ttc tcc      336
   Glu Ala Glu Leu Lys Tyr Asp Pro Pro Phe Gly Phe Arg Lys Phe Ser
               100                 105                 110
   agt aaa gtc cag agc ctc ttg gat atg ctg ccc gaa cat gac ttt tct      384
   Ser Lys Val Gln Ser Leu Leu Asp Met Leu Pro Glu His Asp Phe Ser
           115                 120                 125
   gaa cac ttg aga gcc aag gcc tgc aag cgc tgt gtg gtt gtt ggg aac      432
   Glu His Leu Arg Ala Lys Ala Cys Lys Arg Cys Val Val Val Gly Asn
       130                 135                 140
   ggg ggc atc ctg cac gga cta gag ctg ggt cac gcc ctc aac cag ttc      480
   Gly Gly Ile Leu His Gly Leu Glu Leu Gly His Ala Leu Asn Gln Phe
   145                 150                 155                 160
   gat gtg gta ata agg ttg aac agt gcg cca gtt gag ggt tac tct gaa      528
   Asp Val Val Ile Arg Leu Asn Ser Ala Pro Val Glu Gly Tyr Ser Glu
                           165                     170                     175
   cac gtt ggg aat aaa act act ata agg atg act tac cca gag ggt gcg        576
   His Val Gly Asn Lys Thr Thr Ile Arg Met Thr Tyr Pro Glu Gly Ala
                   180                     185                     190
   cca ctg tcg gac gtt gaa tac tac gcc aat gat ttg ttc gtt act gtt        624
   Pro Leu Ser Asp Val Glu Tyr Tyr Ala Asn Asp Leu Phe Val Thr Val
               195                     200                     205
   tta ttt aag agt gtt gat ttc aag tgg ctt caa gca atg gta aaa aat        672
   Leu Phe Lys Ser Val Asp Phe Lys Trp Leu Gln Ala Met Val Lys Asn
       210                     215                     220
   gaa agc ctg ccc ttt tgg gtt cgc ctc ttc ttt tgg aag caa gtg gca        720
   Glu Ser Leu Pro Phe Trp Val Arg Leu Phe Phe Trp Lys Gln Val Ala
   225                     230                     235                     240
   gaa aaa gtc cca ctc cag cca aag cac ttc agg att ttg aac cca gtt        768
   Glu Lys Val Pro Leu Gln Pro Lys His Phe Arg Ile Leu Asn Pro Val
                   245                     250                     255
   atc atc aaa gaa act gcc ttc gac atc ctt cag tac tca gag cct cag        816
   Ile Ile Lys Glu Thr Ala Phe Asp Ile Leu Gln Tyr Ser Glu Pro Gln
               260                     265                     270
   tca aga ttc tgg ggc cat gat aag aac atc ccc acg atc ggc gtc att        864
   Ser Arg Phe Trp Gly His Asp Lys Asn Ile Pro Thr Ile Gly Val Ile
               275                     280                     285
   gcc gtt gtc ttg gct aca cat ctg tgt gat gaa gtc agc ctg gca ggc        912
   Ala Val Val Leu Ala Thr His Leu Cys Asp Glu Val Ser Leu Ala Gly
       290                     295                     300
   ttt ggc tac gac ctc agt caa ccc agg acc cct ctg cac tac ttt gac        960
   Phe Gly Tyr Asp Leu Ser Gln Pro Arg Thr Pro Leu His Tyr Phe Asp
   305                     310                     315                     320
   agt cag tgc atg ggc gcc atg cac tgg cag gtc atg cac aat gtg acc       1008
   Ser Gln Cys Met Gly Ala Met His Trp Gln Val Met His Asn Val Thr
                   325                     330                     335
   aca gag acc aag ttc ctc ctg aag ctc ctc aag gag ggc gtg gtg gag       1056
   Thr Glu Thr Lys Phe Leu Leu Lys Leu Leu Lys Glu Gly Val Val Glu
               340                     345                     350
   gac ctc agc ggc ggc atc cac tga                                       1080
   Asp Leu Ser Gly Gly Ile His
               355
<210> 4
   <211> 359
   <212> PRT
   <213> Mus musculus
<400> 4
   Met Arg Arg Pro Ser Leu Leu Ile Lys Asp Ile Cys Lys Cys Thr Leu
   1               5                   10                  15
   Val Ala Phe Gly Val Trp Leu Leu Tyr Ile Leu Ile Leu Asn Tyr Thr
               20                  25                  30
   Ala Glu Glu Cys Asp Met Lys Arg Met His Tyr Val Asp Pro Asp Arg
           35                  40                  45
   Ile Lys Arg Ala Gln Ser Tyr Ala Gln Glu Val Leu Gln Lys Glu Cys
       50                  55                  60
   Arg Pro Arg Tyr Ala Lys Thr Ala Met Ala Leu Leu Phe Glu Asp Arg
   65                  70                  75                  80
   Tyr Ser Ile Asn Leu Glu Pro Phe Val Gln Lys Val Pro Thr Ala Ser
                   85                  90                  95
   Glu Ala Glu Leu Lys Tyr Asp Pro Pro Phe Gly Phe Arg Lys Phe Ser
               100                 105                 110
   Ser Lys Val Gln Ser Leu Leu Asp Met Leu Pro Glu His Asp Phe Ser
           115                 120                 125
   Glu His Leu Arg Ala Lys Ala Cys Lys Arg Cys Val Val Val Gly Asn
       130                 135                 140
   Gly Gly Ile Leu His Gly Leu Glu Leu Gly His Ala Leu Asn Gln Phe
   145                 150                 155                 160
   Asp Val Val Ile Arg Leu Asn Ser Ala Pro Val Glu Gly Tyr Ser Glu
                   165                 170                 175
   His Val Gly Asn Lys Thr Thr Ile Arg Met Thr Tyr Pro Glu Gly Ala
               180                 185                 190
   Pro Leu Ser Asp Val Glu Tyr Tyr Ala Asn Asp Leu Phe Val Thr Val
           195                 200                 205
   Leu Phe Lys Ser Val Asp Phe Lys Trp Leu Gln Ala Met Val Lys Asn
       210                 215                 220
   Glu Ser Leu Pro Phe Trp Val Arg Leu Phe Phe Trp Lys Gln Val Ala
   225                 230                 235                 240
   Glu Lys Val Pro Leu Gln Pro Lys His Phe Arg Ile Leu Asn Pro Val
                   245                 250                 255
   Ile Ile Lys Glu Thr Ala Phe Asp Ile Leu Gln Tyr Ser Glu Pro Gln
               260                 265                 270
   Ser Arg Phe Trp Gly His Asp Lys Asn Ile Pro Thr Ile Gly Val Ile
           275                 280                 285
   Ala Val Val Leu Ala Thr His Leu Cys Asp Glu Val Ser Leu Ala Gly
       290                 295                 300
   Phe Gly Tyr Asp Leu Ser Gln Pro Arg Thr Pro Leu His Tyr Phe Asp
   305                 310                 315                 320
   Ser Gln Cys Met Gly Ala Met His Trp Gln Val Met His Asn Val Thr
                   325                 330                 335
   Thr Glu Thr Lys Phe Leu Leu Lys Leu Leu Lys Glu Gly Val Val Glu
               340                 345                 350
   Asp Leu Ser Gly Gly Ile His
   355
<210> 5
   <211> 1080
   <212> DNA
   <213> Mus musculus
<220>
   <221> CDS
   <222> (1) .. (1080)
   <223>
<400> 5
   atg aga aga ccc agc ttg tta ata aaa gac atc tgc aag tgc acg ttg      48
   Met Arg Arg Pro Ser Leu Leu Ile Lys Asp Ile Cys Lys Cys Thr Leu
   1               5                   10                  15
   gtt gca ttt gga gtc tgg ctc ctg tac atc ctc att ttg aat tac acc      96
   Val Ala Phe Gly Val Trp Leu Leu Tyr Ile Leu Ile Leu Asn Tyr Thr
               20                  25                  30
   gct gaa gaa tgt gac atg aaa aga atg cac tat gtg gac cct gac cgg      144
   Ala Glu Glu Cys Asp Met Lys Arg Met His Tyr Val Asp Pro Asp Arg
           35                  40                  45
   ata aag aga gct cag agc tat gct cag gaa gtc ttg cag aag gaa tgt      192
   Ile Lys Arg Ala Gln Ser Tyr Ala Gln Glu Val Leu Gln Lys Glu Cys
       50                  55                  60
   cgg ccc agg tac gcg aag acg gct atg gct ctg tta ttt gag gac agg      240
   Arg Pro Arg Tyr Ala Lys Thr Ala Met Ala Leu Leu Phe Glu Asp Arg
   65                  70                  75                  80
   tac agc atc aac ttg gag cct ttt gtg cag aag gtc ccc acg gcc agt      288
   Tyr Ser Ile Asn Leu Glu Pro Phe Val Gln Lys Val Pro Thr Ala Ser
                   85                  90                  95
   gaa gct gag ctc aag tat gac ccg cct ttt gga ttc cgg aag ttc tcc      336
   Glu Ala Glu Leu Lys Tyr Asp Pro Pro Phe Gly Phe Arg Lys Phe Ser
               100                 105                 110
   agt aaa gtc cag agc ctc ttg gat atg ctg ccc gaa cat gac ttt tct      384
   Ser Lys Val Gln Ser Leu Leu Asp Met Leu Pro Glu His Asp Phe Ser
           115                 120                 125
   gaa cac ttg aga gcc aag gcc tgc aag cgc tgt gtg gtt gtt ggg aac      432
   Glu His Leu Arg Ala Lys Ala Cys Lys Arg Cys Val Val Val Gly Asn
       130                 135                 140
   ggg ggc atc ctg cac gga cta gag ctg ggt cac gcc ctc aac cag ttc      480
   Gly Gly Ile Leu His Gly Leu Glu Leu Gly His Ala Leu Asn Gln Phe
   145                 150                 155                 160
   gat gtg gta ata agg ttg aac agt gcg cca gtt gag ggt tac tct gaa      528
   Asp Val Val Ile Arg Leu Asn Ser Ala Pro Val Glu Gly Tyr Ser Glu
                   165                 170                 175
   cac gtt ggg aat aaa act act ata agg atg act tac cca gag ggt gcg      576
   His Val Gly Asn Lys Thr Thr Ile Arg Met Thr Tyr Pro Glu Gly Ala
               180                 185                 190
   cca ctg tcg gac gtt gaa tac tac gcc aat gat ttg ttc gtt act gtt      624
   Pro Leu Ser Asp Val Glu Tyr Tyr Ala Asn Asp Leu Phe Val Thr Val
           195                 200                 205
   tta ttt aag agt gtt gat ttc aag tgg ctt caa gca atg gta aaa aag      672
   Leu Phe Lys Ser Val Asp Phe Lys Trp Leu Gln Ala Met Val Lys Lys
       210                 215                 220
   gaa agc ctg ccc ttt tgg gtt cgc ctc ttc ttt tgg aag caa gtg gca      720
   Glu Ser Leu Pro Phe Trp Val Arg Leu Phe Phe Trp Lys Gln Val Ala
   225                 230                 235                 240
   gaa aaa gtc cca ctc cag cca aag cac ttc agg att ttg aac cca gtt      768
   Glu Lys Val Pro Leu Gln Pro Lys His Phe Arg Ile Leu Asn Pro Val
                   245                 250                 255
   atc atc aaa gaa act gcc ttc gac atc ctt cag tac tca gag cct cag      816
   Ile Ile Lys Glu Thr Ala Phe Asp Ile Leu Gln Tyr Ser Glu Pro Gln
               260                 265                 270
   tca aga ttc tgg ggc cat gat aag aac atc ccc acg atc ggc gtc att      864
   Ser Arg Phe Trp Gly His Asp Lys Asn Ile Pro Thr Ile Gly Val Ile
           275                 280                 285
   gcc gtt gtc ttg gct aca cat ctg tgt gat gaa gtc agc ctg gca ggc      912
   Ala Val Val Leu Ala Thr His Leu Cys Asp Glu Val Ser Leu Ala Gly
       290                 295                 300
   ttt ggc tac gac ctc agt caa ccc agg acc cct ctg cac tac ttt gac      960
   Phe Gly Tyr Asp Leu Ser Gln Pro Arg Thr Pro Leu His Tyr Phe Asp
   305                 310                 315                 320
   agt cag tgc atg ggc gcc atg cac tgg cag gtc atg cac aat gtg acc     1008
   Ser Gln Cys Met Gly Ala Met His Trp Gln Val Met His Asn Val Thr
                   325                 330                 335
   aca gag acc aag ttc ctc ctg aag ctc ctc aag gag ggc gtg gtg gag     1056
   Thr Glu Thr Lys Phe Leu Leu Lys Leu Leu Lys Glu Gly Val Val Glu
               340                 345                 350
   gac ctc agc ggc ggc atc cac tga                                     1080
   Asp Leu Ser Gly Gly Ile His
           355
<210> 6
   <211> 359
   <212> PRT
   <213> Mus musculus
<400> 6
   Met Arg Arg Pro Ser Leu Leu Ile Lys Asp Ile Cys Lys Cys Thr Leu
   1 5 10 15
   Val Ala Phe Gly Val Trp Leu Leu Tyr Ile Leu Ile Leu Asn Tyr Thr
   20 25 30
   Ala Glu Glu Cys Asp Met Lys Arg Met His Tyr Val Asp Pro Asp Arg
   35 40 45
   Ile Lys Arg Ala Gln Ser Tyr Ala Gln Glu Val Leu Gln Lys Glu Cys
   50 55 60
   Arg Pro Arg Tyr Ala Lys Thr Ala Met Ala Leu Leu Phe Glu Asp Arg
   65 70 75 80
   Tyr Ser Ile Asn Leu Glu Pro Phe Val Gln Lys Val Pro Thr Ala Ser
   85 90 95
   Glu Ala Glu Leu Lys Tyr Asp Pro Pro Phe Gly Phe Arg Lys Phe Ser
   100 105 110
   Ser Lys Val Gln Ser Leu Leu Asp Met Leu Pro Glu His Asp Phe Ser
   115 120 125
   Glu His Leu Arg Ala Lys Ala Cys Lys Arg Cys Val Val Val Gly Asn
   130 135 140
   Gly Gly Ile Leu His Gly Leu Glu Leu Gly His Ala Leu Asn Gln Phe
   145 150 155 160
   Asp Val Val Ile Arg Leu Asn Ser Ala Pro Val Glu Gly Tyr Ser Glu
   165 170 175
   His Val Gly Asn Lys Thr Thr Ile Arg Met Thr Tyr Pro Glu Gly Ala
   180 185 190
   Pro Leu Ser Asp Val Glu Tyr Tyr Ala Asn Asp Leu Phe Val Thr Val
   195 200 205
   Leu Phe Lys Ser Val Asp Phe Lys Trp Leu Gln Ala Met Val Lys Lys
   210 215 220
   Glu Ser Leu Pro Phe Trp Val Arg Leu Phe Phe Trp Lys Gln Val Ala
   225 230 235 240
   Glu Lys Val Pro Leu Gln Pro Lys His Phe Arg Ile Leu Asn Pro Val
   245 250 255
   Ile Ile Lys Glu Thr Ala Phe Asp Ile Leu Gln Tyr Ser Glu Pro Gln
   260 265 270
   Ser Arg Phe Trp Gly His Asp Lys Asn Ile Pro Thr Ile Gly Val Ile
   275 280 285
   Ala Val Val Leu Ala Thr His Leu Cys Asp Glu Val Ser Leu Ala Gly
   290 295 300
   Phe Gly Tyr Asp Leu Ser Gln Pro Arg Thr Pro Leu His Tyr Phe Asp
   305 310 315 320
   Ser Gln Cys Met Gly Ala Met His Trp Gln Val Met His Asn Val Thr
   325 330 335
   Thr Glu Thr Lys Phe Leu Leu Lys Leu Leu Lys Glu Gly Val Val Glu
   340 345 350
   Asp Leu Ser Gly Gly Ile His
   355
<210> 7
   <211> 1080
   <212> DNA
   <213> Mus musculus
<220>
   <22l> CDS
   <222> (1) .. (1080)
   <223>
<400> 7
   atg aga aga ccc agc ttg tta ata aaa gac atc tgc aag tgc acg ttg      48
   Met Arg Arg Pro Ser Leu Leu Ile Lys Asp Ile Cys Lys Cys Thr Leu
   1               5                   10                  15
   gtt gca ttt gga gtc tgg ctc ctg tac atc ctc att ttg aat tac acc      96
   Val Ala Phe Gly Val Trp Leu Leu Tyr Ile Leu Ile Leu Asn Tyr Thr
               20                  25                  30
   gct gaa gaa tgt gac atg aaa aga atg cac tat gtg gac cct gac cgg      144
   Ala Glu Glu Cys Asp Met Lys Arg Met His Tyr Val Asp Pro Asp Arg
           35                  40                  45
   ata aag aga gct cag agc tat gct cag gaa gtc ttg cag aag gaa tgt      192
   Ile Lys Arg Ala Gln Ser Tyr Ala Gln Glu Val Leu Gln Lys Glu Cys
       50                  55                  60
   cgg ccc agg tac gcg aag acg gct atg gct ctg tta ttt gag gac agg      240
   Arg Pro Arg Tyr Ala Lys Thr Ala Met Ala Leu Leu Phe Glu Asp Arg
   65                  70                  75                  80
   tac agc atc aac ttg gag cct ttt gtg cag aag gtc ccc acg gcc agt      288
   Tyr Ser Ile Asn Leu Glu Pro Phe Val Gln Lys Val Pro Thr Ala Ser
                   85                  90                  95
   gaa gct gag ctc aag tat gac ccg cct ttt gga ttc cgg aag ttc tcc      336
   Glu Ala Glu Leu Lys Tyr Asp Pro Pro Phe Gly Phe Arg Lys Phe Ser
               100                 105                 110
   agt aaa gtc cag agc ctc ttg gat atg ctg ccc gaa cat gac ttt tct      384
   Ser Lys Val Gln Ser Leu Leu Asp Met Leu Pro Glu His Asp Phe Ser
           115                 120                 125
   gaa cac ttg aga gcc aag gcc tgc aag cgc tgt gtg gtt gtt ggg aac      432
   Glu His Leu Arg Ala Lys Ala Cys Lys Arg Cys Val Val Val Gly Asn
       130                 135                 140
   ggg ggc atc ctg cac gga cta gag ctg ggt cac gcc ctc aac cag ttc      480
   Gly Gly Ile Leu His Gly Leu Glu Leu Gly His Ala Leu Asn Gln Phe
   145                 150                 155                 160
   gat gtg gta ata agg ttg aac agt gcg cca gtt gag ggt tac tct gaa 528
   Asp Val Val Ile Arg Leu Asn Ser Ala Pro Val Glu Gly Tyr Ser Glu
   165 170 175
   cac gtt ggg aat aaa act act ata agg atg act tac cca gag ggt gcg 576
   His Val Gly Asn Lys Thr Thr Ile Arg Met Thr Tyr Pro Glu Gly Ala
   180 185 190
   cca ctg tcg gac gtt gaa tac tac gcc aat gat ttg ttc gtt act gtt 624
   Pro Leu Ser Asp Val Glu Tyr Tyr Ala Asn Asp Leu Phe Val Thr Val
   195 200 205
   tta ttt aag agt gtt gat ttc aag tgg ctt caa gca atg gta aaa aat 672
   Leu Phe Lys Ser Val Asp Phe Lys Trp Leu Gln Ala Met Val Lys Asn
   210 215 220
   gaa agc ctg ccc ttt tgg gtt cgc ctc ttc ttt tgg aag caa gtg gca 720
   Glu Ser Leu Pro Phe Trp Val Arg Leu Phe Phe Trp Lys Gln Val Ala
   225 230 235 240
   gaa aaa gtc cca ctc cag cca aag cac ttc agg att ttg aac cca gtt 768
   Glu Lys Val Pro Leu Gln Pro Lys His Phe Arg Ile Leu Asn Pro Val
   245 250 255
   atc atc aaa gaa act gcc ttc gac atc ctt cag tac tca gag cct cag 816
   Ile Ile Lys Glu Thr Ala Phe Asp Ile Leu Gln Tyr Ser Glu Pro Gln
   260 265 270
   tca aga ttc tgg ggc cat gat aag aac atc ccc acg atc ggc gtc att 864
   Ser Arg Phe Trp Gly His Asp Lys Asn Ile Pro Thr Ile Gly Val Ile
   275 280 285
   gcc gtt gtc ttg gct aca cat ctg tgt gat gaa gtc agc ctg gca ggc 912
   Ala Val Val Leu Ala Thr His Leu Cys Asp Glu Val Ser Leu Ala Gly
   290 295 300
   ttt ggc tac gac ctc agt caa ccc agg acc cct ctg cac tac ttt gac 960
   Phe Gly Tyr Asp Leu Ser Gln Pro Arg Thr Pro Leu His Tyr Phe Asp
   305 310 315 320
   agt cag tgc atg ggc gcc atg cac tgg cag gtc atg cac aat gtg cag 1008
   Ser Gln Cys Met Gly Ala Met His Trp Gln Val Met His Asn Val Gln
   325 330 335
   aca gag acc aag ttc ctc ctg aag ctc ctc aag gag ggc gtg gtg gag 1056
   Thr Glu Thr Lys Phe Leu Leu Lys Leu Leu Lys Glu Gly Val Val Glu
   340 345 350
   gac ctc agc ggc ggc atc cac tga 1080
   Asp Leu Ser Gly Gly Ile His
   355
<210> 8
   <211> 359
   <212> PRT
   <213> Mus musculus
<400> 8
   Met Arg Arg Pro Ser Leu Leu Ile Lys Asp Ile Cys Lys Cys Thr Leu
   1 5 10 15
   Val Ala Phe Gly Val Trp Leu Leu Tyr Ile Leu Ile Leu Asn Tyr Thr
   20 25 30
   Ala Glu Glu Cys Asp Met Lys Arg Met His Tyr Val Asp Pro Asp Arg
   35 40 45
   Ile Lys Arg Ala Gln Ser Tyr Ala Gln Glu Val Leu Gln Lys Glu Cys
   50 55 60
   Arg Pro Arg Tyr Ala Lys Thr Ala Met Ala Leu Leu Phe Glu Asp Arg
   65 70 75 80
   Tyr Ser Ile Asn Leu Glu Pro Phe Val Gln Lys Val Pro Thr Ala Ser
   85 90 95
   Glu Ala Glu Leu Lys Tyr Asp Pro Pro Phe Gly Phe Arg Lys Phe Ser
   100 105 110
   Ser Lys Val Gln Ser Leu Leu Asp Met Leu Pro Glu His Asp Phe Ser
   115 120 125
   Glu His Leu Arg Ala Lys Ala Cys Lys Arg Cys Val Val Val Gly Asn
   130 135 140
   Gly Gly Ile Leu His Gly Leu Glu Leu Gly His Ala Leu Asn Gln Phe
   145 150 155 160
   Asp Val Val Ile Arg Leu Asn Ser Ala Pro Val Glu Gly Tyr Ser Glu
   165 170 175
   His Val Gly Asn Lys Thr Thr Ile Arg Met Thr Tyr Pro Glu Gly Ala
   180 185 190
   Pro Leu Ser Asp Val Glu Tyr Tyr Ala Asn Asp Leu Phe Val Thr Val
   195 200 205
   Leu Phe Lys Ser Val Asp Phe Lys Trp Leu Gln Ala Met Val Lys Asn
   210 215 220
   Glu Ser Leu Pro Phe Trp Val Arg Leu Phe Phe Trp Lys Gln Val Ala
   225 230 235 240
   Glu Lys Val Pro Leu Gln Pro Lys His Phe Arg Ile Leu Asn Pro Val
   245 250 255
   Ile Ile Lys Glu Thr Ala Phe Asp Ile Leu Gln Tyr Ser Glu Pro Gln
   260 265 270
   Ser Arg Phe Trp Gly His Asp Lys Asn Ile Pro Thr Ile Gly Val Ile
   275 280 285
   Ala Val Val Leu Ala Thr His Leu Cys Asp Glu Val Ser Leu Ala Gly
   290 295 300
   Phe Gly Tyr Asp Leu Ser Gln Pro Arg Thr Pro Leu His Tyr Phe Asp
   305 310 315 320
   Ser Gln Cys Met Gly Ala Met His Trp Gln Val Met His Asn Val Gln
   325 330 335
   Thr Glu Thr Lys Phe Leu Leu Lys Leu Leu Lys Glu Gly Val Val Glu
   340 345 350
   Asp Leu Ser Gly Gly Ile His
   355
<210> 9
   <211> 1080
   <212> DNA
   <213> Mus musculus
<220>
   <221> CDS
   <222> (1) .. (1080)
   <223>
<400> 9
   atg aga aga ccc agc ttg tta ata aaa gac atc tgc aag tgc acg ttg      48
   Met Arg Arg Pro Ser Leu Leu Ile Lys Asp Ile Cys Lys Cys Thr Leu
   1               5                   10                  15
   gtt gca ttt gga gtc tgg ctc ctg tac atc ctc att ttg aat tac acc      96
   Val Ala Phe Gly Val Trp Leu Leu Tyr Ile Leu Ile Leu Asn Tyr Thr
               20                  25                  30
   gct gaa gaa tgt gac atg aaa aga atg cac tat gtg gac cct gac cgg     144
   Ala Glu Glu Cys Asp Met Lys Arg Met His Tyr Val Asp Pro Asp Arg
           35                  40                  45
   ata aag aga gct cag agc tat gct cag gaa gtc ttg cag aag gaa tgt     192
   Ile Lys Arg Ala Gln Ser Tyr Ala Gln Glu Val Leu Gln Lys Glu Cys
       50                  55                  60
   cgg ccc agg tac gcg aag acg gct atg gct ctg tta ttt gag gac agg     240
   Arg Pro Arg Tyr Ala Lys Thr Ala Met Ala Leu Leu Phe Glu Asp Arg
   65                  70                  75                  80
   tac agc atc aac ttg gag cct ttt gtg cag aag gtc ccc acg gcc agt     288
   Tyr Ser Ile Asn Leu Glu Pro Phe Val Gln Lys Val Pro Thr Ala Ser
                   85                  90                  95
   gaa gct gag ctc aag tat gac ccg cct ttt gga ttc cgg aag ttc tcc     336
   Glu Ala Glu Leu Lys Tyr Asp Pro Pro Phe Gly Phe Arg Lys Phe Ser
               100                 105                 110
   agt aaa gtc cag agc ctc ttg gat atg ctg ccc gaa cat gac ttt tct     384
   Ser Lys Val Gln Ser Leu Leu Asp Met Leu Pro Glu His Asp Phe Ser
           115                 120                 125
   gaa cac ttg aga gcc aag gcc tgc aag cgc tgt gtg gtt gtt ggg aac     432
   Glu His Leu Arg Ala Lys Ala Cys Lys Arg Cys Val Val Val Gly Asn
       130                 135                 140
   ggg ggc atc ctg cac gga cta gag ctg ggt cac gcc ctc aac cag ttc     480
   Gly Gly Ile Leu His Gly Leu Glu Leu Gly His Ala Leu Asn Gln Phe
   145 150 155 160
   gat gtg gta ata agg ttg aac agt gcg cca gtt gag ggt tac tct gaa 528
   Asp Val Val Ile Arg Leu Asn Ser Ala Pro Val Glu Gly Tyr Ser Glu
   165 170 175
   cac gtt ggg agc aaa act act ata agg atg act tac cca gag ggt gcg 576
   His Val Gly Ser Lys Thr Thr Ile Arg Met Thr Tyr Pro Glu Gly Ala
   180 185 190
   cca ctg tcg gac gtt gaa tac tac gcc aat gat ttg ttc gtt act gtt 624
   Pro Leu Ser Asp Val Glu Tyr Tyr Ala Asn Asp Leu Phe Val Thr Val
   195 200 205
   tta ttt aag agt gtt gat ttc aag tgg ctt caa gca atg gta aaa aat 672
   Leu Phe Lys Ser Val Asp Phe Lys Trp Leu Gln Ala Met Val Lys Asn
   210 215 220
   gaa agc ctg ccc ttt tgg gtt cgc ctc ttc ttt tgg aag caa gtg gca 720
   Glu Ser Leu Pro Phe Trp Val Arg Leu Phe Phe Trp Lys Gln Val Ala
   225 230 235 240
   gaa aaa gtc cca ctc cag cca aag cac ttc agg att ttg aac cca gtt 768
   Glu Lys Val Pro Leu Gln Pro Lys His Phe Arg Ile Leu Asn Pro Val
   245 250 255
   atc atc aaa gaa act gcc ttc gac atc ctt cag tac tca gag cct cag 816
   Ile Ile Lys Glu Thr Ala Phe Asp Ile Leu Gln Tyr Ser Glu Pro Gln
   260 265 270
   tca aga ttc tgg ggc cat gat aag aac atc ccc acg atc ggc gtc att 864
   Ser Arg Phe Trp Gly His Asp Lys Asn Ile Pro Thr Ile Gly Val Ile
   275 280 285
   gcc gtt gtc ttg gct aca cat ctg tgt gat gaa gtc agc ctg gca ggc 912
   Ala Val Val Leu Ala Thr His Leu Cys Asp Glu Val Ser Leu Ala Gly
   290 295 300
   ttt ggc tac gac ctc agt caa ccc agg acc cct ctg cac tac ttt gac 960
   Phe Gly Tyr Asp Leu Ser Gln Pro Arg Thr Pro Leu His Tyr Phe Asp
   305 310 315 320
   agt cag tgc atg ggc gcc atg cac tgg cag gtc atg cac aat gtg acc 1008
   Ser Gln Cys Met Gly Ala Met His Trp Gln Val Met His Asn Val Thr
   325 330 335
   aca gag acc aag ttc ctc ctg aag ctc ctc aag gag ggc gtg gtg gag 1056
   Thr Glu Thr Lys Phe Leu Leu Lys Leu Leu Lys Glu Gly Val Val Glu
   340 345 350
   gac ctc agc ggc ggc atc cac tga 1080
   Asp Leu Ser Gly Gly Ile His
   355
<210> 10
   <211> 359
   <212> PRT
   <213> Mus musculus
<400> 10
   Met Arg Arg Pro Ser Leu Leu Ile Lys Asp Ile Cys Lys Cys Thr Leu
   1               5                   10                  15
   Val Ala Phe Gly Val Trp Leu Leu Tyr Ile Leu Ile Leu Asn Tyr Thr
               20                  25                  30
   Ala Glu Glu Cys Asp Met Lys Arg Met His Tyr Val Asp Pro Asp Arg
           35                  40                  45
   Ile Lys Arg Ala Gln Ser Tyr Ala Gln Glu Val Leu Gln Lys Glu Cys
       50                  55                  60
   Arg Pro Arg Tyr Ala Lys Thr Ala Met Ala Leu Leu Phe Glu Asp Arg
   65                  70                  75                  80
   Tyr Ser Ile Asn Leu Glu Pro Phe Val Gln Lys Val Pro Thr Ala Ser
                   85                  90                  95
   Glu Ala Glu Leu Lys Tyr Asp Pro Pro Phe Gly Phe Arg Lys Phe Ser
               100                 105                 110
   Ser Lys Val Gln Ser Leu Leu Asp Met Leu Pro Glu His Asp Phe Ser
           115                 120                 125
   Glu His Leu Arg Ala Lys Ala Cys Lys Arg Cys Val Val Val Gly Asn
       130                 135                 140
   Gly Gly Ile Leu His Gly Leu Glu Leu Gly His Ala Leu Asn Gln Phe
   145                 150                 155                 160
   Asp Val Val Ile Arg Leu Asn Ser Ala Pro Val Glu Gly Tyr Ser Glu
                   165                 170                 175
   His Val Gly Ser Lys Thr Thr Ile Arg Met Thr Tyr Pro Glu Gly Ala
               180                 185                 190
   Pro Leu Ser Asp Val Glu Tyr Tyr Ala Asn Asp Leu Phe Val Thr Val
           195                 200                 205
   Leu Phe Lys Ser Val Asp Phe Lys Trp Leu Gln Ala Met Val Lys Asn
       210                 215                 220
   Glu Ser Leu Pro Phe Trp Val Arg Leu Phe Phe Trp Lys Gln Val Ala
   225                 230                 235                 240
   Glu Lys Val Pro Leu Gln Pro Lys His Phe Arg Ile Leu Asn Pro Val
                   245                 250                 255
   Ile Ile Lys Glu Thr Ala Phe Asp Ile Leu Gln Tyr Ser Glu Pro Gln
               260                 265                 270
   Ser Arg Phe Trp Gly His Asp Lys Asn Ile Pro Thr Ile Gly Val Ile
           275                 280                 285
   Ala Val Val Leu Ala Thr His Leu Cys Asp Glu Val Ser Leu Ala Gly
       290                 295                 300
   Phe Gly Tyr Asp Leu Ser Gln Pro Arg Thr Pro Leu His Tyr Phe Asp
   305                 310                 315                 320
   Ser Gln Cys Met Gly Ala Met His Trp Gln Val Met His Asn Val Thr
                   325                 330                 335
   Thr Glu Thr Lys Phe Leu Leu Lys Leu Leu Lys Glu Gly Val Val Glu
   340 345 350
   Asp Leu Ser Gly Gly Ile His
   355
<210> 11
   <211> 1080
   <212> DNA
   <213> Mus musculus
<220>
   <221> CDS
   <222> (1) .. (1080)
   <223>
<400> 11
   atg aga aga ccc agc ttg tta ata aaa gac atc tgc aag tgc acg ttg 48
   Met Arg Arg Pro Ser Leu Leu Ile Lys Asp Ile Cys Lys Cys Thr Leu
   1 5 10 15
   gtt gca ttt gga gtc tgg ctc ctg tac atc ctc att ttg aat tac acc 96
   Val Ala Phe Gly Val Trp Leu Leu Tyr Ile Leu Ile Leu Asn Tyr Thr
   20 25 30
   gct gaa gaa tgt gac atg aaa aga atg cac tat gtg gac cct gac cgg 144
   Ala Glu Glu Cys Asp Met Lys Arg Met His Tyr Val Asp Pro Asp Arg
   35 40 45
   ata aag aga gct cag agc tat gct cag gaa gtc ttg cag aag gaa tgt 192
   Ile Lys Arg Ala Gln Ser Tyr Ala Gln Glu Val Leu Gln Lys Glu Cys
   50 55 60
   cgg ccc agg tac gcg aag acg gct atg gct ctg tta ttt gag gac agg 240
   Arg Pro Arg Tyr Ala Lys Thr Ala Met Ala Leu Leu Phe Glu Asp Arg
   65 70 75 80
   tac agc atc aac ttg gag cct ttt gtg cag aag gtc ccc acg gcc agt 288
   Tyr Ser Ile Asn Leu Glu Pro Phe Val Gln Lys Val Pro Thr Ala Ser
   85 90 95
   gaa gct gag ctc aag tat gac ccg cct ttt gga ttc cgg aag ttc tcc 336
   Glu Ala Glu Leu Lys Tyr Asp Pro Pro Phe Gly Phe Arg Lys Phe Ser
   100 105 110
   agt aaa gtc cag agc ctc ttg gat atg ctg ccc gaa cat gac ttt tct 384
   Ser Lys Val Gln Ser Leu Leu Asp Met Leu Pro Glu His Asp Phe Ser
   115 120 125
   gaa cac ttg aga gcc aag gcc tgc aag cgc tgt gtg gtt gtt ggg aac 432
   Glu His Leu Arg Ala Lys Ala Cys Lys Arg Cys Val Val Val Gly Asn
   130 135 140
   ggg ggc atc ctg cac gga cta gag ctg ggt cac gcc ctc aac cag ttc 480
   Gly Gly Ile Leu His Gly Leu Glu Leu Gly His Ala Leu Asn Gln Phe
   145 150 155 160
   gat gtg gta ata agg ttg aac agt gcg cca gtt gag ggt tac tct gaa 528
   Asp Val Val Ile Arg Leu Asn Ser Ala Pro Val Glu Gly Tyr Ser Glu
   165 170 175
   gac gtt ggg agc aaa act act ata agg atg act tac cca gag ggt gcg 576
   Asp Val Gly Ser Lys Thr Thr Ile Arg Met Thr Tyr Pro Glu Gly Ala
   180 185 190
   cca ctg tcg gac gtt gaa tac tac gcc aat gat ttg ttc gtt act gtt 624
   Pro Leu Ser Asp Val Glu Tyr Tyr Ala Asn Asp Leu Phe Val Thr Val
   195 200 205
   tta ttt aag agt gtt gat ttc aag tgg ctt caa gca atg gta aaa aat 672
   Leu Phe Lys Ser Val Asp Phe Lys Trp Leu Gln Ala Met Val Lys Asn
   210 215 220
   gaa agc ctg ccc ttt tgg gtt cgc ctc ttc ttt tgg aag caa gtg gca 720
   Glu Ser Leu Pro Phe Trp Val Arg Leu Phe Phe Trp Lys Gln Val Ala
   225 230 235 240
   gaa aaa gtc cca ctc cag cca aag cac ttc agg att ttg aac cca gtt 768
   Glu Lys Val Pro Leu Gln Pro Lys His Phe Arg Ile Leu Asn Pro Val
   245 250 255
   atc atc aaa gaa act gcc ttc gac atc ctt cag tac tca gag cct cag 816
   Ile Ile Lys Glu Thr Ala Phe Asp Ile Leu Gln Tyr Ser Glu Pro Gln
   260 265 270
   tca aga ttc tgg ggc cat gat aag aac atc ccc acg atc ggc gtc att 864
   Ser Arg Phe Trp Gly His Asp Lys Asn Ile Pro Thr Ile Gly Val Ile
   275 280 285
   gcc gtt gtc ttg gct aca cat ctg tgt gat gaa gtc agc ctg gca ggc 912
   Ala Val Val Leu Ala Thr His Leu Cys Asp Glu Val Ser Leu Ala Gly
   290 295 300
   ttt ggc tac gac ctc agt caa ccc agg acc cct ctg cac tac ttt gac 960
   Phe Gly Tyr Asp Leu Ser Gln Pro Arg Thr Pro Leu His Tyr Phe Asp
   305 310 315 320
   agt cag tgc atg ggc gcc atg cac tgg cag gtc atg cac aat gtg acc 1008
   Ser Gln Cys Met Gly Ala Met His Trp Gln Val Met His Asn Val Thr
   325 330 335
   aca gag acc aag ttc ctc ctg aag ctc ctc aag gag ggc gtg gtg gag 1056
   Thr Glu Thr Lys Phe Leu Leu Lys Leu Leu Lys Glu Gly Val Val Glu
   340 345 350
   gac ctc agc ggc ggc atc cac tga 1080
   Asp Leu Ser Gly Gly Ile His
   355
<210> 12
   <211> 359
   <212> PRT
   <213> Mus musculus
<400> 12
   Met Arg Arg Pro Ser Leu Leu Ile Lys Asp Ile Cys Lys Cys Thr Leu
   1               5                   10                  15
   Val Ala Phe Gly Val Trp Leu Leu Tyr Ile Leu Ile Leu Asn Tyr Thr
               20                  25                  30
   Ala Glu Glu Cys Asp Met Lys Arg Met His Tyr Val Asp Pro Asp Arg
           35                  40                  45
   Ile Lys Arg Ala Gln Ser Tyr Ala Gln Glu Val Leu Gln Lys Glu Cys
       50                  55                  60
   Arg Pro Arg Tyr Ala Lys Thr Ala Met Ala Leu Leu Phe Glu Asp Arg
   65                  70                  75                  80
   Tyr Ser Ile Asn Leu Glu Pro Phe Val Gln Lys Val Pro Thr Ala Ser
                   85                  90                  95
   Glu Ala Glu Leu Lys Tyr Asp Pro Pro Phe Gly Phe Arg Lys Phe Ser
               100                 105                 110
   Ser Lys Val Gln Ser Leu Leu Asp Met Leu Pro Glu His Asp Phe Ser
           115                 120                 125
   Glu His Leu Arg Ala Lys Ala Cys Lys Arg Cys Val Val Val Gly Asn
       130                 135                 140
   Gly Gly Ile Leu His Gly Leu Glu Leu Gly His Ala Leu Asn Gln Phe
   145                 150                 155                 160
   Asp Val Val Ile Arg Leu Asn Ser Ala Pro Val Glu Gly Tyr Ser Glu
                   165                 170                 175
   Asp Val Gly Ser Lys Thr Thr Ile Arg Met Thr Tyr Pro Glu Gly Ala
               180                 185                 190
   Pro Leu Ser Asp Val Glu Tyr Tyr Ala Asn Asp Leu Phe Val Thr Val
           195                 200                 205
   Leu Phe Lys Ser Val Asp Phe Lys Trp Leu Gln Ala Met Val Lys Asn
       210                 215                 220
   Glu Ser Leu Pro Phe Trp Val Arg Leu Phe Phe Trp Lys Gln Val Ala
   225                 230                 235                 240
   Glu Lys Val Pro Leu Gln Pro Lys His Phe Arg Ile Leu Asn Pro Val
                   245                 250                 255
   Ile Ile Lys Glu Thr Ala Phe Asp Ile Leu Gln Tyr Ser Glu Pro Gln
               260                 265                 270
   Ser Arg Phe Trp Gly His Asp Lys Asn Ile Pro Thr Ile Gly Val Ile
           275                 280                 285
   Ala Val Val Leu Ala Thr His Leu Cys Asp Glu Val Ser Leu Ala Gly
       290                 295                 300
   Phe Gly Tyr Asp Leu Ser Gln Pro Arg Thr Pro Leu His Tyr Phe Asp
   305                 310                 315                 320
   Ser Gln Cys Met Gly Ala Met His Trp Gln Val Met His Asn Val Thr
   325 330 335
   Thr Glu Thr Lys Phe Leu Leu Lys Leu Leu Lys Glu Gly Val Val Glu
   340 345 350
   Asp Leu Ser Gly Gly Ile His
   355
<210> 13
   <211> 1080
   <212> DNA
   <213> Mus musculus
<220>
   <221> CDS
   <222> (1) .. (1080)
   <223>
<400> 13
   atg aga aga ccc agc ttg tta ata aaa gac atc tgc aag tgc acg ttg 48
   Met Arg Arg Pro Ser Leu Leu Ile Lys Asp Ile Cys Lys Cys Thr Leu
   1 5 10 15
   gtt gca ttt gga gtc tgg ctc ctg tac atc ctc att ttg aat tac acc 96
   Val Ala Phe Gly Val Trp Leu Leu Tyr Ile Leu Ile Leu Asn Tyr Thr
   20 25 30
   gct gaa gaa tgt gac atg aaa aga atg cac tat gtg gac cct gac cgg 144
   Ala Glu Glu Cys Asp Met Lys Arg Met His Tyr Val Asp Pro Asp Arg
   35 40 45
   ata aag aga gct cag agc tat gct cag gaa gtc ttg cag aag gaa tgt 192
   Ile Lys Arg Ala Gln Ser Tyr Ala Gln Glu Val Leu Gln Lys Glu Cys
   50 55 60
   cgg ccc agg tac gcg aag acg gct atg gct ctg tta ttt gag gac agg 240
   Arg Pro Arg Tyr Ala Lys Thr Ala Met Ala Leu Leu Phe Glu Asp Arg
   65 70 75 80
   tac agc atc aac ttg gag cct ttt gtg cag aag gtc ccc acg gcc agt 288
   Tyr Ser Ile Asn Leu Glu Pro Phe Val Gln Lys Val Pro Thr Ala Ser
   85 90 95
   gaa gct gag ctc aag tat gac ccg cct ttt gga ttc cgg aag ttc tcc 336
   Glu Ala Glu Leu Lys Tyr Asp Pro Pro Phe Gly Phe Arg Lys Phe Ser
   100 105 110
   agt aaa gtc cag agc ctc ttg gat atg ctg ccc gaa cat gac ttt tct 384
   Ser Lys Val Gln Ser Leu Leu Asp Met Leu Pro Glu His Asp Phe Ser
   115 120 125
   gaa cac ttg aga gcc aag gcc tgc aag cgc tgt gtg gtt gtt ggg aac 432
   Glu His Leu Arg Ala Lys Ala Cys Lys Arg Cys Val Val Val Gly Asn
   130 135 140
   ggg ggc atc ctg cac gga cta gag ctg ggt cac gcc ctc aac cag ttc 480
   Gly Gly Ile Leu His Gly Leu Glu Leu Gly His Ala Leu Asn Gln Phe
   145 150 155 160
   gat gtg gta ata agg ttg aac agt gcg cca gtt gag ggt tac tct gaa 528
   Asp Val Val Ile Arg Leu Asn Ser Ala Pro Val Glu Gly Tyr Ser Glu
   165 170 175
   gac gtt ggg aat aaa act act ata agg atg act tac cca gag ggt gcg 576
   Asp Val Gly Asn Lys Thr Thr Ile Arg Met Thr Tyr Pro Glu Gly Ala
   180 185 190
   cca ctg tcg gac gtt gaa tac tac gcc aat gat ttg ttc gtt act gtt 624
   Pro Leu Ser Asp Val Glu Tyr Tyr Ala Asn Asp Leu Phe Val Thr Val
   195 200 205
   tta ttt aag agt gtt gat ttc aag tgg ctt caa gca atg gta aaa aat 672
   Leu Phe Lys Ser Val Asp Phe Lys Trp Leu Gln Ala Met Val Lys Asn
   210 215 220
   gaa agc ctg ccc ttt tgg gtt cgc ctc ttc ttt tgg aag caa gtg gca 720
   Glu Ser Leu Pro Phe Trp Val Arg Leu Phe Phe Trp Lys Gln Val Ala
   225 230 235 240
   gaa aaa gtc cca ctc cag cca aag cac ttc agg att ttg aac cca gtt 768
   Glu Lys Val Pro Leu Gln Pro Lys His Phe Arg Ile Leu Asn Pro Val
   245 250 255
   atc atc aaa gaa act gcc ttc gac atc ctt cag tac tca gag cct cag 816
   Ile Ile Lys Glu Thr Ala Phe Asp Ile Leu Gln Tyr Ser Glu Pro Gln
   260 265 270
   tca aga ttc tgg ggc cat gat aag aac atc ccc acg atc ggc gtc att 864
   Ser Arg Phe Trp Gly His Asp Lys Asn Ile Pro Thr Ile Gly Val Ile
   275 280 285
   gcc gtt gtc ttg gct aca cat ctg tgt gat gaa gtc agc ctg gca ggc 912
   Ala Val Val Leu Ala Thr His Leu Cys Asp Glu Val Ser Leu Ala Gly
   290 295 300
   ttt ggc tac gac ctc agt caa ccc agg acc cct ctg cac tac ttt gac 960
   Phe Gly Tyr Asp Leu Ser Gln Pro Arg Thr Pro Leu His Tyr Phe Asp
   305 310 315 320
   agt cag tgc atg ggc gcc atg cac tgg cag gtc atg cac aat gtg acc 1008
   Ser Gln Cys Met Gly Ala Met His Trp Gln Val Met His Asn Val Thr
   325 330 335
   aca gag acc aag ttc ctc ctg aag ctc ctc aag gag ggc gtg gtg gag 1056
   Thr Glu Thr Lys Phe Leu Leu Lys Leu Leu Lys Glu Gly Val Val Glu
   340 345 350
   gac ctc agc ggc ggc atc cac tga 1080
   Asp Leu Ser Gly Gly Ile His
   355
<210> 14
   <211> 359
   <212> PRT
   <213> Mus musculus
<400> 14
   Met Arg Arg Pro Ser Leu Leu Ile Lys Asp Ile Cys Lys Cys Thr Leu
   1 5 10 15
   Val Ala Phe Gly Val Trp Leu Leu Tyr Ile Leu Ile Leu Asn Tyr Thr
   20 25 30
   Ala Glu Glu Cys Asp Met Lys Arg Met His Tyr Val Asp Pro Asp Arg
   35 40 45
   Ile Lys Arg Ala Gln Ser Tyr Ala Gln Glu Val Leu Gln Lys Glu Cys
   50 55 60
   Arg Pro Arg Tyr Ala Lys Thr Ala Met Ala Leu Leu Phe Glu Asp Arg
   65 70 75 80
   Tyr Ser Ile Asn Leu Glu Pro Phe Val Gln Lys Val Pro Thr Ala Ser
   85 90 95
   Glu Ala Glu Leu Lys Tyr Asp Pro Pro Phe Gly Phe Arg Lys Phe Ser
   100 105 110
   Ser Lys Val Gln Ser Leu Leu Asp Met Leu Pro Glu His Asp Phe Ser
   115 120 125
   Glu His Leu Arg Ala Lys Ala Cys Lys Arg Cys Val Val Val Gly Asn
   130 135 140
   Gly Gly Ile Leu His Gly Leu Glu Leu Gly His Ala Leu Asn Gln Phe
   145 150 155 160
   Asp Val Val Ile Arg Leu Asn Ser Ala Pro Val Glu Gly Tyr Ser Glu
   165 170 175
   Asp Val Gly Asn Lys Thr Thr Ile Arg Met Thr Tyr Pro Glu Gly Ala
   180 185 190
   Pro Leu Ser Asp Val Glu Tyr Tyr Ala Asn Asp Leu Phe Val Thr Val
   195 200 205
   Leu Phe Lys Ser Val Asp Phe Lys Trp Leu Gln Ala Met Val Lys Asn
   210 215 220
   Glu Ser Leu Pro Phe Trp Val Arg Leu Phe Phe Trp Lys Gln Val Ala
   225 230 235 240
   Glu Lys Val Pro Leu Gln Pro Lys His Phe Arg Ile Leu Asn Pro Val
   245 250 255
   Ile Ile Lys Glu Thr Ala Phe Asp Ile Leu Gln Tyr Ser Glu Pro Gln
   260 265 270
   Ser Arg Phe Trp Gly His Asp Lys Asn Ile Pro Thr Ile Gly Val Ile
   275 280 285
   Ala Val Val Leu Ala Thr His Leu Cys Asp Glu Val Ser Leu Ala Gly
   290 295 300
   Phe Gly Tyr Asp Leu Ser Gln Pro Arg Thr Pro Leu His Tyr Phe Asp
   305 310 315 320
   Ser Gln Cys Met Gly Ala Met His Trp Gln Val Met His Asn Val Thr
                       325                 330                 335
   Thr Glu Thr Lys Phe Leu Leu Lys Leu Leu Lys Glu Gly Val Val Glu
                   340                 345                 350
   Asp Leu Ser Gly Gly Ile His
               355
<210> 15
   <211> 1080
   <212> DNA
   <213> Mus musculus
<220>
   <221> CDS
   <222> (1) .. (1080)
   <223>
<400> 15
   atg aga aga ccc agc ttg tta ata aaa gac atc tgc aag tgc acg ttg      48
   Met Arg Arg Pro Ser Leu Leu Ile Lys Asp Ile Cys Lys Cys Thr Leu
   1               5                   10                  15
   gtt gca ttt gga gtc tgg ctc ctg tac atc ctc att ttg aat tac acc      96
   Val Ala Phe Gly Val Trp Leu Leu Tyr Ile Leu Ile Leu Asn Tyr Thr
               20                  25                  30
   gct gaa gaa tgt gac atg aaa aga atg cac tat gtg gac cct gac cgg     144
   Ala Glu Glu Cys Asp Met Lys Arg Met His Tyr Val Asp Pro Asp Arg
           35                  40                  45
   ata aag aga gct cag agc tat gct cag gaa gtc ttg cag aag gaa tgt     192
   Ile Lys Arg Ala Gln Ser Tyr Ala Gln Glu Val Leu Gln Lys Glu Cys
       50                  55                  60
   cgg ccc agg tac gcg aag acg gct atg gct ctg tta ttt gag gac agg     240
   Arg Pro Arg Tyr Ala Lys Thr Ala Met Ala Leu Leu Phe Glu Asp Arg
   65                  70                  75                  80
   tac agc atc aac ttg gag cct ttt gtg cag aag gtc ccc acg gcc agt     288
   Tyr Ser Ile Asn Leu Glu Pro Phe Val Gln Lys Val Pro Thr Ala Ser
                   85                  90                  95
   gaa gct gag ctc aag tat gac ccg cct ttt gga ttc cgg aag ttc tcc     336
   Glu Ala Glu Leu Lys Tyr Asp Pro Pro Phe Gly Phe Arg Lys Phe Ser
               100                 105                 110
   agt aaa gtc cag agc ctc ttg gat atg ctg ccc gaa cat gac ttt tct     384
   Ser Lys Val Gln Ser Leu Leu Asp Met Leu Pro Glu His Asp Phe Ser
           115                 120                 125
   gaa cac ttg aga gcc aag gcc tgc aag cgc tgt gtg gtt gtt ggg aac     432
   Glu His Leu Arg Ala Lys Ala Cys Lys Arg Cys Val Val Val Gly Asn
                 130                     135                     140
   ggg ggc atc ctg cac gga cta gag ctg ggt cac gcc ctc aac cag ttc       480
   Gly Gly Ile Leu His Gly Leu Glu Leu Gly His Ala Leu Asn Gln Phe
   145                     150                     155                 160
   gat gtg gta ata agg ttg aac agt gcg cca gtt gag ggt tac tct gaa       528
   Asp Val Val Ile Arg Leu Asn Ser Ala Pro Val Glu Gly Tyr Ser Glu
                       165                     170                 175
   gac gtt ggg agc aaa act act ata agg atg act tac cca gag ggt gcg       576
   Asp Val Gly Ser Lys Thr Thr Ile Arg Met Thr Tyr Pro Glu Gly Ala
                   180                     185                 190
   cca ctg tcg gac gtt gaa tac tac gcc aat gat ttg ttc gtt act gtt       624
   Pro Leu Ser Asp Val Glu Tyr Tyr Ala Asn Asp Leu Phe Val Thr Val
               195                     200                 205
   tta ttt aag agt gtt gat ttc aag tgg ctt caa gca atg gta aaa aag       672
   Leu Phe Lys Ser Val Asp Phe Lys Trp Leu Gln Ala Met Val Lys Lys
       210                     215                 220
   gaa agc ctg ccc ttt tgg gtt cgc ctc ttc ttt tgg aag caa gtg gca       720
   Glu Ser Leu Pro Phe Trp Val Arg Leu Phe Phe Trp Lys Gln Val Ala
   225                     230                     235                 240
   gaa aaa gtc cca ctc cag cca aag cac ttc agg att ttg aac cca gtt       768
   Glu Lys Val Pro Leu Gln Pro Lys His Phe Arg Ile Leu Asn Pro Val
                       245                     250                 255
   atc atc aaa gaa act gcc ttc gac atc ctt cag tac tca gag cct cag       816
   Ile Ile Lys Glu Thr Ala Phe Asp Ile Leu Gln Tyr Ser Glu Pro Gln
                   260                     265                 270
   tca aga ttc tgg ggc cat gat aag aac atc ccc acg atc ggc gtc att       864
   Ser Arg Phe Trp Gly His Asp Lys Asn Ile Pro Thr Ile Gly Val Ile
               275                     280                 285
   gcc gtt gtc ttg gct aca cat ctg tgt gat gaa gtc agc ctg gca ggc       912
   Ala Val Val Leu Ala Thr His Leu Cys Asp Glu Val Ser Leu Ala Gly
       290                     295                 300
   ttt ggc tac gac ctc agt caa ccc agg acc cct ctg cac tac ttt gac       960
   Phe Gly Tyr Asp Leu Ser Gln Pro Arg Thr Pro Leu His Tyr Phe Asp
   305                     310                     315                 320
   agt cag tgc atg ggc gcc atg cac tgg cag gtc atg cac aat gtg cag      1008
   Ser Gln Cys Met Gly Ala Met His Trp Gln Val Met His Asn Val Gln
                       325                     330                 335
   aca gag acc aag ttc ctc ctg aag ctc ctc aag gag ggc gtg gtg gag      1056
   Thr Glu Thr Lys Phe Leu Leu Lys Leu Leu Lys Glu Gly Val Val Glu
                   340                     345                 350
   gac ctc agc ggc ggc atc cac tga                                      1080
   Asp Leu Ser Gly Gly Ile His
               355
<210> 16
   <211> 359
   <212> PRT
   <213> Mus musculus
<400> 16
   Met Arg Arg Pro Ser Leu Leu Ile Lys Asp Ile Cys Lys Cys Thr Leu
   1               5                   10                  15
   Val Ala Phe Gly Val Trp Leu Leu Tyr Ile Leu Ile Leu Asn Tyr Thr
               20                  25                  30
   Ala Glu Glu Cys Asp Met Lys Arg Met His Tyr Val Asp Pro Asp Arg
           35                  40                  45
   Ile Lys Arg Ala Gln Ser Tyr Ala Gln Glu Val Leu Gln Lys Glu Cys
       50                  55                  60
   Arg Pro Arg Tyr Ala Lys Thr Ala Met Ala Leu Leu Phe Glu Asp Arg
   65                  70                  75                  80
   Tyr Ser Ile Asn Leu Glu Pro Phe Val Gln Lys Val Pro Thr Ala Ser
                   85                  90                  95
   Glu Ala Glu Leu Lys Tyr Asp Pro Pro Phe Gly Phe Arg Lys Phe Ser
               100                 105                 110
   Ser Lys Val Gln Ser Leu Leu Asp Met Leu Pro Glu His Asp Phe Ser
           115                 120                 125
   Glu His Leu Arg Ala Lys Ala Cys Lys Arg Cys Val Val Val Gly Asn
       130                 135                 140
   Gly Gly Ile Leu His Gly Leu Glu Leu Gly His Ala Leu Asn Gln Phe
   145                 150                 155                 160
   Asp Val Val Ile Arg Leu Asn Ser Ala Pro Val Glu Gly Tyr Ser Glu
                   165                 170                 175
   Asp Val Gly Ser Lys Thr Thr Ile Arg Met Thr Tyr Pro Glu Gly Ala
               180                 185                 190
   Pro Leu Ser Asp Val Glu Tyr Tyr Ala Asn Asp Leu Phe Val Thr Val
           195                 200                 205
   Leu Phe Lys Ser Val Asp Phe Lys Trp Leu Gln Ala Met Val Lys Lys
       210                 215                 220
   Glu Ser Leu Pro Phe Trp Val Arg Leu Phe Phe Trp Lys Gln Val Ala
   225                 230                 235                 240
   Glu Lys Val Pro Leu Gln Pro Lys His Phe Arg Ile Leu Asn Pro Val
                   245                 250                 255
   Ile Ile Lys Glu Thr Ala Phe Asp Ile Leu Gln Tyr Ser Glu Pro Gln
               260                 265                 270
   Ser Arg Phe Trp Gly His Asp Lys Asn Ile Pro Thr Ile Gly Val Ile
           275                 280                 285
   Ala Val Val Leu Ala Thr His Leu Cys Asp Glu Val Ser Leu Ala Gly
       290                 295                 300
   Phe Gly Tyr Asp Leu Ser Gln Pro Arg Thr Pro Leu His Tyr Phe Asp
   305 310 315 320
   Ser Gln Cys Met Gly Ala Met His Trp Gln Val Met His Asn Val Gln
   325 330 335
   Thr Glu Thr Lys Phe Leu Leu Lys Leu Leu Lys Glu Gly Val Val Glu
   340 345 350
   Asp Leu Ser Gly Gly Ile His
   355
<210> 17
   <211> 1080
   <212> DNA
   <213> Mus musculus
<220>
   <221> CDS
   <222> (1) .. (1080)
   <223>
<400> 17
   atg aga aga ccc agc ttg tta ata aaa gac atc tgc aag tgc acg ttg 48
   Met Arg Arg Pro Ser Leu Leu Ile Lys Asp Ile Cys Lys Cys Thr Leu
   1 5 10 15
   gtt gca ttt gga gtc tgg ctc ctg tac atc ctc att ttg aat tac acc 96
   Val Ala Phe Gly Val Trp Leu Leu Tyr Ile Leu Ile Leu Asn Tyr Thr
   20 25 30
   gct gaa gaa tgt gac atg aaa aga atg cac tat gtg gac cct gac cgg 144
   Ala Glu Glu Cys Asp Met Lys Arg Met His Tyr Val Asp Pro Asp Arg
   35 40 45
   ata aag aga gct cag agc tat gct cag gaa gtc ttg cag aag gaa tgt 192
   Ile Lys Arg Ala Gln Ser Tyr Ala Gln Glu Val Leu Gln Lys Glu Cys
   50 55 60
   cgg ccc agg tac gcg aag acg gct atg gct ctg tta ttt gag gac agg 240
   Arg Pro Arg Tyr Ala Lys Thr Ala Met Ala Leu Leu Phe Glu Asp Arg
   65 70 75 80
   tac agc atc aac ttg gag cct ttt gtg cag aag gtc ccc acg gcc agt 288
   Tyr Ser Ile Asn Leu Glu Pro Phe Val Gln Lys Val Pro Thr Ala Ser
   85 90 95
   gaa gct gag ctc aag tat gac ccg cct ttt gga ttc cgg aag ttc tcc 336
   Glu Ala Glu Leu Lys Tyr Asp Pro Pro Phe Gly Phe Arg Lys Phe Ser
   100 105 110
   agt aaa gtc cag agc ctc ttg gat atg ctg ccc gaa cat gac ttt tct 384
   Ser Lys Val Gln Ser Leu Leu Asp Met Leu Pro Glu His Asp Phe Ser
   115 120 125
   gaa cac ttg aga gcc aag gcc tgc aag cgc tgt gtg gtt gtt ggg aac 432
   Glu His Leu Arg Ala Lys Ala Cys Lys Arg Cys Val Val Val Gly Asn
       130                 135                 140
   ggg ggc atc ctg cac gga cta gag ctg ggt cac gcc ctc aac cag ttc      480
   Gly Gly Ile Leu His Gly Leu Glu Leu Gly His Ala Leu Asn Gln Phe
   145                 150                 155                 160
   gat gtg gta ata agg ttg aac agt gcg cca gtt gag ggt tac tct gaa      528
   Asp Val Val Ile Arg Leu Asn Ser Ala Pro Val Glu Gly Tyr Ser Glu
                   165                 170                 175
   cac gtt ggg aat aaa act act ata agg atg act tac cca gag ggt gcg      576
   His Val Gly Asn Lys Thr Thr Ile Arg Met Thr Tyr Pro Glu Gly Ala
               180                 185                 190
   cca ctg tcg gac gtt gaa tac tac gcc aat gat ttg ttc gtt act gtt      624
   Pro Leu Ser Asp Val Glu Tyr Tyr Ala Asn Asp Leu Phe Val Thr Val
           195                 200                 205
   tta ttt aag agt gtt gat ttc aag tgg ctt caa gca atg gta aaa aag      672
   Leu Phe Lys Ser Val Asp Phe Lys Trp Leu Gln Ala Met Val Lys Lys
       210                 215                 220
   gaa agc ctg ccc ttt tgg gtt cgc ctc ttc ttt tgg aag caa gtg gca      720
   Glu Ser Leu Pro Phe Trp Val Arg Leu Phe Phe Trp Lys Gln Val Ala
   225                 230                 235                 240
   gaa aaa gtc cca ctc cag cca aag cac ttc agg att ttg aac cca gtt      768
   Glu Lys Val Pro Leu Gln Pro Lys His Phe Arg Ile Leu Asn Pro Val
                   245                 250                 255
   atc atc aaa gaa act gcc ttc gac atc ctt cag tac tca gag cct cag      816
   Ile Ile Lys Glu Thr Ala Phe Asp Ile Leu Gln Tyr Ser Glu Pro Gln
               260                 265                 270
   tca aga ttc tgg ggc cat gat aag aac atc ccc acg atc ggc gtc att      864
   Ser Arg Phe Trp Gly His Asp Lys Asn Ile Pro Thr Ile Gly Val Ile
           275                 280                 285
   gcc gtt gtc ttg gct aca cat ctg tgt gat gaa gtc agc ctg gca ggc      912
   Ala Val Val Leu Ala Thr His Leu Cys Asp Glu Val Ser Leu Ala Gly
       290                 295                 300
   ttt ggc tac gac ctc agt caa ccc agg acc cct ctg cac tac ttt gac      960
   Phe Gly Tyr Asp Leu Ser Gln Pro Arg Thr Pro Leu His Tyr Phe Asp
   305                 310                 315                 320
   agt cag tgc atg ggc gcc atg cac tgg cag gtc atg cac aat gtg cag     1008
   Ser Gln Cys Met Gly Ala Met His Trp Gln Val Met His Asn Val Gln
                   325                 330                 335
   aca gag acc aag ttc ctc ctg aag ctc ctc aag gag ggc gtg gtg gag     1056
   Thr Glu Thr Lys Phe Leu Leu Lys Leu Leu Lys Glu Gly Val Val Glu
               340                 345                 350
   gac ctc agc ggc ggc atc cac tga                                     1080
   Asp Leu Ser Gly Gly Ile His
           355
<210> 18
   <211> 359
   <212> PRT
   <213> Mus musculus
<400> 18
   Met Arg Arg Pro Ser Leu Leu Ile Lys Asp Ile Cys Lys Cys Thr Leu
   1               5                   10                  15
   Val Ala Phe Gly Val Trp Leu Leu Tyr Ile Leu Ile Leu Asn Tyr Thr
               20                  25                  30
   Ala Glu Glu Cys Asp Met Lys Arg Met His Tyr Val Asp Pro Asp Arg
           35                  40                  45
   Ile Lys Arg Ala Gln Ser Tyr Ala Gln Glu Val Leu Gln Lys Glu Cys
       50                  55                  60
   Arg Pro Arg Tyr Ala Lys Thr Ala Met Ala Leu Leu Phe Glu Asp Arg
   65                  70                  75                  80
   Tyr Ser Ile Asn Leu Glu Pro Phe Val Gln Lys Val Pro Thr Ala Ser
                   85                  90                  95
   Glu Ala Glu Leu Lys Tyr Asp Pro Pro Phe Gly Phe Arg Lys Phe Ser
               100                 105                 110
   Ser Lys Val Gln Ser Leu Leu Asp Met Leu Pro Glu His Asp Phe Ser
           115                 120                 125
   Glu His Leu Arg Ala Lys Ala Cys Lys Arg Cys Val Val Val Gly Asn
       130                 135                 140
   Gly Gly Ile Leu His Gly Leu Glu Leu Gly His Ala Leu Asn Gln Phe
   145                 150                 155                 160
   Asp Val Val Ile Arg Leu Asn Ser Ala Pro Val Glu Gly Tyr Ser Glu
                   165                 170                 175
   His Val Gly Asn Lys Thr Thr Ile Arg Met Thr Tyr Pro Glu Gly Ala
               180                 185                 190
   Pro Leu Ser Asp Val Glu Tyr Tyr Ala Asn Asp Leu Phe Val Thr Val
           195                 200                 205
   Leu Phe Lys Ser Val Asp Phe Lys Trp Leu Gln Ala Met Val Lys Lys
       210                 215                 220
   Glu Ser Leu Pro Phe Trp Val Arg Leu Phe Phe Trp Lys Gln Val Ala
   225                 230                 235                 240
   Glu Lys Val Pro Leu Gln Pro Lys His Phe Arg Ile Leu Asn Pro Val
                   245                 250                 255
   Ile Ile Lys Glu Thr Ala Phe Asp Ile Leu Gln Tyr Ser Glu Pro Gln
               260                 265                 270
   Ser Arg Phe Trp Gly His Asp Lys Asn Ile Pro Thr Ile Gly Val Ile
           275                 280                 285
   Ala Val Val Leu Ala Thr His Leu Cys Asp Glu Val Ser Leu Ala Gly
       290                 295                 300
   Phe Gly Tyr Asp Leu Ser Gln Pro Arg Thr Pro Leu His Tyr Phe Asp
   305 310 315 320
   Ser Gln Cys Met Gly Ala Met His Trp Gln Val Met His Asn Val Gln
   325 330 335
   Thr Glu Thr Lys Phe Leu Leu Lys Leu Leu Lys Glu Gly Val Val Glu
   340 345 350
   Asp Leu Ser Gly Gly Ile His
   355
<210> 19
   <211> 34
   <212> DNA
   <213> Artificial
<220>
   <223> Description of Artificial Sequence: primer for N180Q mutagenesis
<400> 19
   ctctgaacac gttgggcaga aaactactat aagg 34
<210> 20
   <2l1> 34
   <212> DNA ,
   <213> Artificial
<220>
   <223> Description of Artificial Sequence: primer for N180Q mutagenesis
<400> 20
   ccttatagta gttttctgcc caacgtgttc agag 34
<210> 21
   <211> 34
   <212> DNA
   <213> Artificial
<220>
   <223> Description of Artificial Sequence: primer for N180K mutagenesis
<400> 21
   ctctgaacac gttgggaaga aaactactat aagg 34
<210> 22
   <211> 34
   <212> DNA
   <213> Artificial
<220>
   <223> Description of Artificial Sequence: primer for N180K mutagenesis
<400> 22
   ccttatagta gttttcttcc caacgtgttc agag 34
<210> 23
   <211> 34
   <212> DNA
   <213> Artificial
<220>
   <223> Description of Artificial Sequence: primer for N180S mutagenesis
<400> 23
   ctctgaacac gttgggagca aaactactat aagg 34
<210> 24
   <211> 34
   <212> DNA
   <213> Artificial
<220>
   <223> Description of Artificial Sequence: primer for N180S mutagenesis
<400> 24
   ccttatagta gttttgctcc caacgtgttc agag 34
<210> 25
   <211> 42
   <212> DNA
   <213> Artificial
<220>
   <223> Description of Artificial Sequence: primer for H177D, N180S mutagenesis
<400> 25
   gagggttact ctgaagacgt tgggagcaaa actactataa gg 42
<210> 26
   <211> 42
   <212> DNA
   <213> Artificial
<220>
   <223> Description of Artificial Sequence: primer for H177D, N180S mutagenesis
<400> 26
   ccttatagta gttttgctcc caacgtcttc agagtaaccc tc 42
<210> 27
   <211> 35
   <212> DNA
   <213> Artificial
<220>
   <223> Description of Artificial Sequence: primer for H177D mutagenesis
<400> 27
   gagggttact ctgaagacgt tgggaataaa actac 35
<210> 28
   <211> 35
   <212> DNA
   <213> Artificial
<220>
   <223> Description of Artificial Sequence: primer for H177D mutagenesis
<400> 28
   gtagttttat tcccaacgtc ttcagagtaa ccctc 35
<210> 29
   <211> 27
   <212> DNA
   <213> Artificial
<220>
   <223> Description of Artificial Sequence: primer for N224Q mutagenesis
<400> 29
   gcaatggtaa aacaggaaag cctgccc 27
<210> 30
   <211> 27
   <212> DNA
   <213> Artificial
<220>
   <223> Description of Artificial Sequence: primer for N224Q mutagenesis
<400> 30
   gggcaggctt tcctgtttta ccattgc 27
<210> 31
   <211> 27
   <212> DNA
   <213> Artificial
<220>
   <223> Description of Artificial Sequence: primer for N224K mutagenesis
<400> 31
   gcaatggtaa aaaaggaaag cctgccc 27
<210> 32
   <211> 27
   <212> DNA
   <213> Artificial
<220>
   <223> Description of Artificial Sequence: primer for N224K mutagenesis
<400> 32
   gggcaggctt tcctttttta ccattgc 27
<210> 33
   <211> 39
   <212> DNA
   <213> Artificial
<220>
   <223> Description of Artificial Sequence: primer for N224D mutagenesis
<400> 33
   gcttcaagca atggtaaaag atgaaagcct gcccttttg 39
<210> 34
   <211> 39
   <212> DNA
   <213> Artificial
<220>
   <223> Description of Artificial Sequence: primer for N224D mutagenesis
<400> 34
   caaaagggca ggctttcatc ttttaccatt gcttgaagc 39
<210> 35
   <211> 37
   <212> DNA
   <213> Artificial
<220>
   <223> Description of Artificial Sequence: primer for N334Q mutagenesis
<400> 35
   ctggcaggtc atgcaccagg tgaccacaga gaccaag 37
<210> 36
   <211> 37
   <212> DNA
   <213> Artificial
<220>
   <223> Description of Artificial Sequence: primer for N334Q mutagenesis
<400> 36
   cttggtctct gtggtcacct tgtgcatgac ctgccag 37
<210> 37
   <211> 37
   <212> DNA
   <213> Artificial
<220>
   <223> Description of Artificial Sequence: primer for N334K mutagehesis
<400> 37
   cttggtctct gtggtcacct tgtgcatgac ctgccag 37
<210> 38
   <211> 37
   <212> DNA
   <213> Artificial
<220>
   <223> Description of Artificial Sequence: primer for N334K mutagenesis
<400> 38
   cttggtctct gtggtcacct tgtgcatgac ctgccag 37
<210> 39
   <211> 39
   <212> DNA
   <213> Artificial
<220>
   <223> Description of Artificial Sequence: primer for T336Q mutagenesis
<400> 39
   caggtcatgc acaatgtgca gacagagacc aagttcctc 39
<210> 40
   <211> 39
   <212> DNA
   <213> Artificial
<220>
   <223> Description of Artificial Sequence: primer for T336Q mutagenesis
<400> 40 39
   gaggaacttg gtctctgtct gcacattgtg catgacctg 39
<210> 41
   <211> 364
   <212> PRT
   <213> Danio rerio
<400> 41
   Met Arg Arg Val Met Lys Gln Ser Ser Cys Tyr Phe Ser Lys Arg Thr
   1 5 10 15
   Met Ile Leu Leu Leu Ser Leu Ala Leu Met Ser Leu Ala Phe Leu Lys
   20 25 30
   Leu Pro Ser Phe His Thr Glu Leu Lys Pro Val Glu Val Pro Val Asp
   35 40 45
   Asn Lys Phe Arg Lys Arg Val His Ser His Val Arg Glu Ile Leu Asp
   50 55 60
   Lys Glu Cys Arg Pro Ser Phe Ala Arg Gln Arg Met Val Thr Glu His
   65 70 75 80
   His Gly Ser Thr Pro Thr Ile Asp Pro Phe Leu Asn Lys Asn Met Lys
   85 90 95
   Leu Asp Glu Gln Ile Phe Gln Tyr Pro Pro Pro Phe Gly Phe Leu Asp
   100 105 110
   Met Lys Lys Lys Leu Glu Glu Ile Leu Asn Leu Leu Pro Val Ser Ser
   115 120 125
   Glu Gln Arg Leu Gly Glu Arg Asp Cys Arg Arg Cys Val Val Val Gly
   130 135 140
   Asn Gly Gly Ile Leu Lys Gly Leu Gly Leu Gly His Leu Leu Asn Arg
   145 150 155 160
   Phe Asp Ile Ile Ile Arg Leu Asn Ser Gly Pro Leu Gln Asp Phe Ser
   165 170 175
   Ala Asp Val Gly Asn Arg Thr Thr Ile Arg Met Ser Tyr Pro Glu Ser
   180 185 190
   Cys Pro Lys Val Trp Glu Asp Thr Asp Pro Asp Leu Lys Tyr Val Ala
   195 200 205
   Val Ile Phe Lys Ser Val Asp Phe His Trp Leu Arg Ala Met Ile Ser
   210 215 220
   Arg Thr Pro Val Ser Leu Trp Asp Arg Leu Phe Phe Trp Gln Asn Val
   225 230 235 240
   Pro Met Ser Val Pro Val Lys Thr Ser Gln Phe His Leu Leu Asn Pro
   245 250 255
   Gln Ile Ile Arg Glu Met Ala Leu Asp Leu Leu Asn Tyr Pro Glu Pro
   260 265 270
   Lys Lys Arg Leu Trp Ser Trp Asp Gln Asn Ile Pro Thr Leu Gly Leu
   275 280 285
   Thr Ala Leu Asn Leu Ala Thr Tyr Ile Cys Asp Glu Val Ser Leu Ala
   290 295 300
   Gly Phe Gly Tyr Asn Leu Ser Gln Lys Glu Ala Pro Leu His Tyr Tyr
   305 310 315 320
   Asp Ser Val Pro Met Thr Thr Ile Leu Lys Glu Ala Met His Asn Val
   325 330 335
   Gln Lys Glu Thr Val Phe Leu Lys Arg Leu Val Ala Ser Gly Ser Ile
   340 345 350
   Thr Asp Leu Thr Gly Gly Ile His Cys Ser Phe Cys
   355 360
<210> 42
   <211> 362
   <212> PRT
   <213> Homo sapiens
<400> 42
   Met Arg Arg Pro Ser Leu Leu Leu Lys Asp Ile Leu Lys Cys Thr Leu
   1 5 10 15
   Leu Val Phe Gly Val Trp Ile Leu Tyr Ile Leu Lys Leu Asn Tyr Thr
   20 25 30
   Thr Glu Glu Cys Asp Met Lys Lys Met His Tyr Val Asp Pro Asp His
   35 40 45
   Val Lys Arg Ala Gln Lys Tyr Ala Gln Gln Val Leu Gln Lys Glu Cys
   50 55 60
   Arg Pro Lys Phe Ala Lys Thr Ser Met Ala Leu Leu Phe Glu His Arg
   65 70 75 80
   Tyr Ser Val Asp Leu Leu Pro Phe Val Gln Lys Ala Pro Lys Asp Ser
   85 90 95
   Glu Ala Glu Ser Lys Tyr Asp Pro Pro Phe Gly Phe Arg Lys Phe Ser
   100 105 110
   Ser Lys Val Gln Thr Leu Leu Glu Leu Leu Pro Glu His Asp Leu Pro
   115 120 125
   Glu His Leu Lys Ala Lys Thr Cys Arg Arg Cys Val Val Ile Gly Ser
   130 135 140
   Gly Gly Ile Leu His Gly Leu Glu Leu Gly His Thr Leu Asn Gln Phe
   145 150 155 160
   Asp Val Val Ile Arg Leu Asn Ser Ala Pro Val Glu Gly Tyr Ser Glu
   165 170 175
   His Val Gly Asn Lys Thr Thr Ile Arg Met Thr Tyr Pro Glu Gly Ala
   180 185 190
   Pro Leu Ser Asp Leu Glu Tyr Tyr Ser Asn Asp Leu Phe Val Ala Val
   195 200 205
   Leu Phe Lys Ser Val Asp Phe Asn Trp Leu Gln Ala Met Val Lys Lys
   210 215 220
   Glu Thr Leu Pro Phe Trp Val Arg Leu Phe Phe Trp Lys Gln Val Ala
   225 230 235 240
   Glu Lys Ile Pro Leu Gln Pro Lys His Phe Arg Ile Leu Asn Pro Val
   245 250 255
   Ile Ile Lys Glu Thr Ala Phe Asp Ile Leu Gln Tyr Ser Glu Pro Gln
   260 265 270
   Ser Arg Phe Trp Gly Arg Asp Lys Asn Val Pro Thr Ile Gly Val Ile
   275 280 285
   Ala Val Val Leu Ala Thr His Leu Cys Asp Glu Val Ser Leu Ala Gly
   290 295 300
   Phe Gly Tyr Asp Leu Asn Gln Pro Arg Thr Pro Leu His Tyr Phe Asp
   305 310 315 320
   Ser Gln Cys Met Ala Ala Met Asn Phe Gln Thr Met His Asn Val Thr
   325 330 335
   Thr Glu Thr Lys Phe Leu Leu Lys Leu Val Lys Glu Gly Val Val Lys
   340 345 350
   Asp Leu Ser Gly Gly Ile Asp Arg Glu Phe
   355 360
<210> 43
   <211> 6
   <212> PRT
   <213> Mus musculus
<400> 43

## Description

### Technical Field

The present invention relates to a mutant protein of an asparagine-linked glycoprotein, which has no N-linked sugar chain and retains a physiological activity of a glycoprotein before the mutation was introduced, and a method of producing the same.

### Background Art

First, abbreviations used in the specification are explained.
BPB: bromophenol blue
CMP: cytidine 5'-monophosphate
EDTA: ethylenediamine tetraacetic acid
EGTA: ethylene glycol bis(2-aminoehtyl ether)-N,N,N',N'-tetraacetic acid
Endo H: endo-β-N-acetylglucosaminidase H
ER: endoplasmic reticulum
FBS: fetal bovine serum
HRP: horseradish peroxidase
HPTLC: high performance thin-layer chromatography
LacCer: lactosylceramide
N-linked sugar chain: asparagine-linked sugar chain
N-linked glycoprotein: asparagine-linked glycoprotein
PBS: phosphate-buffered saline
PCR: polymerase chain reaction
PMSF: phenylmethanesulfonyl fluoride
PNGase F: peptide-N(4)-(N-acetyl-β-D-glucosaminyl) asparagine amidase
PVDF: polyvinylidene difluoride
SAT-I: sialyltransferase-I
hSAT-I: human SAT-I
mSAT-I: mouse SAT-I
zSAT-I: zebrafish SAT-I
SDS: sodium dodecyl sulfate
SDS-PAGE: sodium dodecyl sulfate-polyacrylamide electrophoresis
Further, in the specification, an amino acid X (one-letter notation) which is at the J-th position from the N-terminal of a protein is represented as "XJ". For example, Asn at the 180th position from the N-terminal is represented as "N 180" and Thr at the 336th position from the N-terminal is represented as "T336".

Further, in the specification, a protein obtained by substituting an amino acid X (one-letter notation) at the J-th position from the N-terminal of a protein by an amino acid Z (one-letter notation) is represented as "XJZ". For example, a protein in which His at the 177th position from the N-terminal is substituted by Asp is represented as "H177D" and a protein in which Asn at the 224th position from the N-terminal is substituted by Lys is represented as "N224K". Further, when substitution of one amino acid by another occurs at a plurality of sites, the substitutions are represented side by side. For example, a protein in which His at the 177th position from the N-terminal is substituted by Asp and Asn at the 224th position from the N-terminal is substituted by Lys is represented as "H177D, N224K".

Most of secretory proteins in a living body are considered to be present as glycoproteins. Sugar chains have a wide variety of functions such as physical stabilization of proteins, expression of enzyme activity, cell adhesion, metastasis of cancer, signal transduction, subcellular localization, microbial infection, and immune response. Therefore, lack of the sugar chains to be linked to glycoproteins, in many cases, causes effects such as failure or reduction of expression of physiological activity of the glycoprotein.

An N-linked sugar chain, which is a sugar chain to be linked to a glycoprotein, is known to link to Asn in a consensus sequence comprising Asn-Xaa-Ser/Thr (provided that Xaa is an amino acid other than Pro) of a protein.

It is also known that a glycosyltransferase is one of glycoproteins, and a plurality of N-linked sugar chains are linked to most of glycosyltransferases. It has been reported that a glycosyltransferase wherein N in said consensus sequence is substituted by Q, which is structurally most similar to N, and a glycosyltransferase treated with tunicamycin, which inhibits formation of dolichol pyrophosphate-N-acetylglucosamine to suppress sugar transfer to a protein, have a remarkably reduced enzymatic activity (Non-patent Document 1 and Non-patent Document 2).

For example, α1,3-fucosyltransferase (Fuc) III, IV, V and VI, α2,3-sialyltransferase (ST3Gal I), α2,6-sialyltransferase (ST6Gal I), α2,8-sialyltransferase (ST8Sia I); GD3 synthase, UDP-N-acetylglucosamine:β-D-mannoside β1,4-N-acetylglucosaminyltransferase III (GnT III), core 2β1,6-N-acetylglucosaminyltransferase (C2 GnT), galactosylceramide sulfotransferase (CST), N-acetylgalactosaminyltransferase 1 (GalNac-T), β1,3-galactosyltransferase (Gal-T2), UDP-glucuronosyltransferase 2B (UGT2B) and the like are reported to require sugar chains to express their enzymatic activities.

SAT-I is one of sialyltransferases (i.e., sialic acid transferases) that transfer sialic acid to lactosylceramide and synthesizes GM3, and hSAT-I, mSAT-I and zSAT-I have been cloned (Non-patent Document 3 and Non-patent Document 4). In addition, SAT-Is from dog, bovine, rat, chiken, medaka, and tetradon have been cloned.

However, in N-linked glycoproteins, a mutant protein is not known which has no N-linked sugar chains to be linked thereto, but retains a physiological activity of a glycoprotein before the mutation is introduced. Neither is a method of producing such a mutant protein and the like.

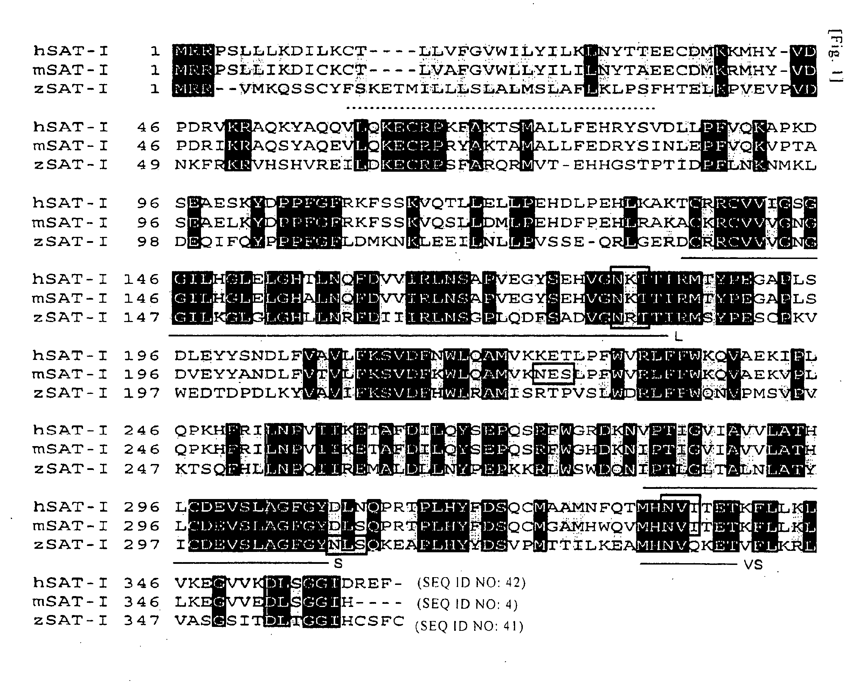

Non-patent Document 1: Martina J. A. et al., 1998, The Journal of Biological Chemistry, vol. 273, p. 3725-3731
Non-patent Document 2: Eckhardt M. et al., 2002, The Biochemical Journal vol. 368, p. 317-324
Non-patent Document 3: Ishii A. et al., 1998, The Journal of Biological Chemistry, vol. 273, p. 31652-31655
Non-patent Document 4: Kono M. et al., 1998, Biochemical and Biophysical Research Communications, vol. 253, p. 170-175
Non -patent Document 5: Jeanneau et al., 2004, Journal of Biological Chemistry, vol 279, p.13461-13468, discloses mutants of a human sialyltransferase (hST3Gal I) in the conserved N glycosylation motif N-X-S/T, in particular, N114S (NDT to SDT), N201 Q (NVS to QVS)and N79D (NQT to DQT). The motif is the same as in the present application and was identified by comparing its sequence with other related glycosyltransferases, namely, sialyltransferases (e.g. fig. 1). The mutations N79D and N114S destroy the N-glycosylation sites of the sialyltransferase but when glycosyltransferase activity is measured, it does not destroy enzymatic activity (p. 13465).
Non -patent Document 6: Chen C. et al., 2000, Glycobiology, vol. 10, p.531-583 discloses mutants of a sialyltransferase (ST6Gal I) in the conserved motif N-X-S/T (same as motif (i) of the application) of N146S (NVS to SVS) and N158Q (NTT to QTT). The N146S mutation reduces the activity of the enzyme from 41 to 28 sialyltransferase units/pmol and hour, but did not alter its folding (p 532-533, table I).
Non -patent Document 7: Close. B. et al., 2000, Journal of Biological Chemistry, vol. 275, p. 4484-4491, discloses mutants of a sialyltransferase (ST8Sia IV) in the conserved N - glycosylation motif N-X-S/T (motif I), namely, N to S at residues 50, 74, 119, 191, 219, 327. Some of these mutations maintain enzymatic activity (discussion, p. 4486, fig. 1). In particular, when looking at the sequence of ST8Sia IV, the mutation N204S is from NES to SES.
Non -patent Document 8: Uemura, S. et al (2003), Glycobiology, vol. 13, p. 855-856 (Abstract of the Annual Conference of the Society of Glycobiology), discloses the impact of mutations in the N-glycosylation sites N-X-T/S (motif I) on murine SAT-I, N180Q, N224Q, N334Q, which result in mutants retaining 10-30% of the activity of the wild type.

### Disclosure of the Invention

If a physiological activity of a glycoprotein is expressed in a state where a sugar chain to be linked to the glycoprotein is absent, a protein which has the physiological activity equivalent to that of the glycoprotein can be produced conveniently, rapidly, in large amounts and at low cost and its quality can be kept constant. Therefore, it is an object of the present invention to provide a mutant protein of an N-linked glycoprotein, which lacks an N-linked sugar chain to be linked, but retains the physiological activity of the glycoprotein, and to provide a method of producing the same.

The inventors of the present invention made an intensive effort to achieve the above-mentioned objects and as a result, they have found that substitution of an amino acid in a specified amino acid sequence in the polypeptide of an N-linked glycoprotein by other amino acid allows the glycoprotein to retain the function of the glycoprotein before the mutation is introduced even in a state where N-linked sugar chains are absent, thereby accomplishing the present invention.

Thus, the present invention provides a mutant protein (hereinafter, referred to as "the protein of the present invention") as defined in claims 1 or 3.

Further, the glycoprotein is a sialyltransferase.
Further, the mutant SAT-1 is, in particular, preferably a protein having the following amino acid sequence.
· the amino acid sequence of SEQ ID NO: 6.
· the amino acid sequence of SEQ ID NO: 8.
· the amino acid sequence of SEQ ID NO: 10.
· the amino acid sequence of SEQ ID NO: 12.
· the amino acid sequence of SEQ ID NO: 14.
· the amino acid sequence of SEQ ID NO: 16.
· the amino acid sequence of SEQ ID NO: 18.

Further, the present invention provides a polynucleotide encoding the protein of the present invention (hereinafter, referred to as "the polynucleotide of the present invention").

### Brief Description of the Drawings

Fig. 1 is a drawing showing a comparison among amino acid sequences of hSAT-I, mSAT-I, and zSAT I. In Fig. 1, the dotted crossbar line portion indicates a transmembrane region, the first solid crossbar line portion indicates a sialyl motif L, the second solid crossbar line portion indicates a sialyl motif S, and the third solid crossbar line portion indicates a sialyl motif VS. A boxed portion indicates the portion to which an N-linked sugar chain can be linked (i.e., N-glycosylation site).
Fig. 2 is a diagram (photograph) showing immunoblotting of cell lysates expressing mSAT-I.
Fig. 3 is a diagram (photograph) showing immunoblotting of cell lysates expressing mSAT-I in the presence of tunicamycin (Tuni.), Kifnecin (Kif.), or castanospermine (Cas.).
Fig. 4 is a diagram showing the SAT-I activity of the lysate used in Fig. 3. "*" indicates a significant difference with respect to the wild type at p<0.001.
Fig. 5 is a diagram (photograph) showing immunoblotting of cell lysates of expressing the mSAT-I mutant.
Fig. 6 is a diagram (photograph) showing immunoblotting of Endo H-treated cell lysates expressing the mSAT-I mutant.
Fig. 7 is a diagram (photograph) showing immunoblotting of PNGase F-treated cell lysates expressing the mSAT-I mutant.
Fig. 8 is a diagram showing results of Fig. 7 (i.e., total amount of SAT-I) in numerical values. "*" indicates a significant difference with respect to the wild type at p<0.05.
Fig. 9 is a diagram (photograph) showing sensitivity of the mSAT-I mutant to trypsin.
Fig. 10 is a diagram (photograph) showing the enzyme activity of the mSAT-I mutant. "*" indicates a significant difference with respect to the wild type at p<0.001.
Fig. 11 is a diagram (photograph) showing immunoblotting of cell lysates of expressing mSAT-I mutants.
Fig. 12 is a diagram showing the enzyme activity of the mSAT-I mutants. "*" indicates a significant difference with respect to the wild type at p<0.001.
Fig. 13 is a diagram (photograph) showing immunoblotting of cell lysates expressing mSAT-I mutants.
Fig. 14 is a diagram showing the enzyme activity of the mSAT-I mutants. "*" indicates a significant difference with respect to the wild type at p<0.001 and "**" indicates a significant difference with respect to the wild type at p<0.05.
Fig. 15 is a diagram (photograph) showing immunoblotting of cell lysates of expressing mSAT-1 mutants.
Fig. 16 is a diagram showing the enzyme activity of the mSAT-1 mutants. "*" indicates a significant difference with respect to the wild type at p<0.001 and "**" indicates a significant difference with respect to the wild type at p<0.05.
Fig. 17 is a diagram (photograph) showing immunoblotting of cell lysates expressing mSAT-1 mutants.
Fig. 18 is a diagram showing the enzyme activity of the mSAT-1 mutant. "*" indicates a significant difference with respect to the wild type at p<0.001.
Fig. 19 is a diagram (photograph) showing immunoblotting of a lysate and culture medium of HEK293 cells which express mSAT-I . "1" to "4" indicate vectors used as controls, "5" to "8" show results of mSAT-I, "C" and "M" show results of a lysate of each cell and culture medium, respectively, PNGase F(-) indicates results without PNGase F treatment, and PNGase F(+) indicates results with PNGase F treatment.
Fig. 20 is a diagram showing the secretion amount of the mSAT-I mutant. Note that the secretion amount is indicated as a ratio of the amount of SAT-I protein secreted to a substrate to the amount of SAT-I protein present in the cell. "*" indicates a significant difference with respect to the wild type at p<0.001 and "**" indicates a significant difference with respect to the wild type at p<0.02.
Fig. 21 is a diagram (photograph) showing the enzyme activities of mSAT-I, "H177D, N180S, N224K, T336Q" mutants and the wild type mSAT-I which are expressed in E.coli. "-" in the first lane from the left indicates buffer only, and the first and second lanes from the right indicate 0.05 µg and 0.25 µg of GM3 standard, respectively.

### Description of the Preferred Enbodiment

Hereinafter, detailed description will be made with reference to embodiments of the present invention.

### <1> protein of the present invention

The protein of the present invention is a mutant protein comprising an amino acid sequence ofN-linked glycoprotein as defined in claims 1 and 3.

In the present invention, the glycoprotein into which a mutation is introduced is preferably one which has some physiological activities. The protein of the present invention is characterized by retaining the physiological activity of the original glycoprotein even if it does not retain a part of or all the N-linked sugar chains to be linked. Whether the physiological activity of the glycoprotein is retained can be examined by comparing it with the physiological activity of the glycoprotein before the mutation is introduced by using a method of measuring the physiological activity that is selected as appropriate by one skilled in the art depending on the kind of the glycoprotein.
The phase "retains a physiological activity" does not necessarily mean that the mutant glycoprotein must have the same or higher activity than that of the N-linked glycoprotein before the mutation is introduced, as far as the mutant glycoprotein has the same kind of a physiological activity as the N-linked glycoprotein before the mutation is introduced. However, it is preferable that the mutant protein have preferably 30% or more activity, more preferably 50% or more activity than that of the N-linked glycoprotein before the mutation is introduced.
The amino acid sequence of SAT-I before the mutation is introduced is not particularly limited as far as it serves as an enzyme that transfers sialic acid to lactosylceramide to synthesize GM3, that is a sialyltransferase that catalyzes a reaction wherein sialic acid is transferred to non-reducing terminal galactose through α-2,3-linkage.
Further, the mutant SAT-1 is particularly preferably the protein having the following amino acid sequences.
· the amino acid sequence of SEQ ID NO: 6.
· the amino acid sequence of SEQ ID NO: 8.
· the amino acid sequence of SEQ ID NO: 10.
· the amino acid sequence of SEQ ID NO: 12.
· the amino acid sequence of SEQ ID NO: 14.
· the amino acid sequence of SEQ ID NO: 16.
· the amino acid sequence of SEQ ID NO: 18.

In addition, other proteins (including peptide or peptides) may be linked to the protein of the present invention as far as the proteins give no substantial influence to the physiological activity of the protein of the present invention. That is, the term "protein of the present invention" includes a fusion protein composed of the protein of the present invention to which one or more other proteins (including peptide or peptides) is/are linked. The term "other proteins" as used herein includes peptides, and the kind of the other protein is not particularly limited and may be selected as appropriate depending on the purpose. Production of a fusion protein is intended to, for example, allow the protein to be secreted outside the cells, facilitate separation and purification, facilitate detection, or combine activities of a plurality of proteins. However, the object is not limited thereto. Reference is made to the explanation regarding the section "Polynucleotide of the Present Invention" described hereinbelow about examples of the term "other proteins".

The protein of the present invention can be either chemically synthesized or produced by genetic engineering since it has been clarified by the present invention which part of it should be substituted and how it should be made. Regarding the method of producing the protein of the present invention by genetic engineering, reference is made to the section "Polynucleotide of the Present Invention" and Examples described hereinbelow.
Whether the protein of the present invention is obtained, synthesized, produced or the like can be readily judged by analyzing the amino acid sequence (or nucleotide sequence of the polynucleotide encoding the same) of a resultant protein and comparing the amino acid sequence with the sequence of the protein of the present invention (or the polynucleotide of the present invention).
Further, in the protein of the present invention, whether an N-linked sugar chain is linked to the amino acid sequence that contains the substituted amino acids can be conveniently examined by observing whether a sugar chain is released and whether the molecular weight of the protein is changed by allowing a suitable glycosidase (for example, PNGase F) to react with the protein.

### <2> Polynucleotide of the Present Invention

The polynucleotide of the present invention is a polynucleotide that encodes the protein of the present invention.

The polynucleotide that encodes the protein of the present invention includes various polynucleotides having different nucleotide sequences due to degeneration of a genetic code.
One skilled in the art can easily understand that any of such polynucleotides is included in the polynucleotide of the present invention.

For example, to obtain the mutant mSAT-I having amino acid sequences set forth below by introducing a mutation into mSAT-I having the amino acid sequence of SEQ ID NO: 4, the polynucleotides having the following nucleotide sequences may be used. However, it can be readily understood that these polynucleotides are exemplary and the present invention is not limited thereto.

### (Protein)

· the amino acid sequence of SEQ ID NO: 6.
· the amino acid sequence of SEQ ID NO: 8.
· the amino acid sequence of SEQ ID NO: 10.
· the amino acid sequence of SEQ ID NO: 12.
· the amino acid sequence of SEQ ID NO: 14.
· the amino acid sequence of SEQ ID NO: 16.
· the amino acid sequence of SEQ ID NO: 18.

### (Polynucleotide)

· the nucleotide sequence of SEQ ID NO: 5.
· the nucleotide sequence of SEQ ID NO: 7.
· the nucleotide sequence of SEQ ID NO: 9.
· the nucleotide sequence of SEQ ID NO: 11.
· the nucleotide sequence of SEQ ID NO: 13.
· the nucleotide sequence of SEQ ID NO: 15.
· the nucleotide sequence of SEQ ID NO: 17.
Although the nucleotides that constitute the polynucleotide of the present invention may be either a DNA or an RNA, it is preferably a DNA in terms of stability.

The polynucleotides of the present invention includes not only polynucleotides which directly encode the protein of the present invention but also polynucleotides having a sequence complementary to them.

The polynucleotide of the present invention can be produced, for example, by the following method.
First, the amino acid sequence of N-linked glycoprotein of interest is analyzed. Also, the nucleotide sequence of a polynucleotide that encodes the N-linked glycoprotein is determined as necessary. Then, nucleotides of a corresponding portion of the polynucleotide that encodes the glycoprotein is substituted by other nucleotides so that at least one amino acid of one or more of the amino acid sequence motif (I) and/or (II) present in the amino acid sequence is substituted by other amino acid(s):
(I) Asn Xa1 Xa2 (SEQ ID NO: 1); and
(II) Xa3 Val Gly Asn Xa Xa2 (SEQ ID NO: 2)
(in (I) and (II), Xa1 indicates an amino acid other than Pro, Xa2 indicates Thr or Ser, and Xa3 indicates His or Asp).

The number and kind of the nucleotide to be substituted are not particularly limited as far as the amino acid to be substituted can be substituted by other amino acid as a target. For example, there may be a case where substitution of only one nucleotide is sufficient or where three nucleotides must be substituted to effect the substitution by the target amino acid. How to introduce substitution of nucleotides can be determined as appropriate by one skilled in the art.

Since it has now been clarified what a portion and how a substitution should be performed, the substitution of nucleotides (i.e., production of the polynucleotide of the present invention) can be performed by using either a technique of chemical synthesis or a technique of genetic engineering.

In the case where the technique of chemical synthesis is used, chemical synthesis may be performed by designing a nucleotide sequence such that the substitution of interest can be introduced and then by performing chemical synthesis so that a nucleotide sequence as designed can be obtained.

In the case where the technique of genetic engineering is used, various methods can be adapted. For example, there can be used a method in which a primer corresponding to a portion of the polynucleotide encoding a target N-linked glycoprotein into which a nucleotide substitution is intended to be introduced (i.e., a primer having a nucleotide substituted by the target nucleotide) is synthesized, and a nucleotide is substituted using a polynucleotide encoding the target glycoprotein as a template and a commercially available mutation introducing kit (for example, QuickChange site-directed mutagenesis kit manufactured by Stratagene). For specific examples thereof, reference is made to the Examples below.

The obtained polynucleotide of the present invention may be further amplified or purified. Also, it may be incorporated into suitable plasmids, vectors, and so on.
The polynucleotide of the present invention thus obtained can be used in the production of the proteins of the present invention.

For example, PCR is performed by using the polynucleotide (i.e., DNA) of the present invention as a template to amplify the polynucleotide of the present invention. Then, the amplified polynucleotide of the present invention is incorporated into a suitable expression vector or the like.
The vector to be used herein is not particularly limited and can be selected as appropriate by one skilled in the art depending on the kind of cells (i.e., host) into which a gene is introduced and so on. For example, when eukaryotic cells are used as host cells, an expression vector for eukaryotic cells can be selected, while when prokaryotic cells are used as host cells, an expression vector for prokaryotic cells can be selected.

In particular, the expression vector for eukaryotic cells are preferably used, and an expression vector for mammalian cells are more preferably used.

Further, the expression vector may be constructed so that the protein of the present invention encoded by the polynucleotide of the present invention can be isolated and purified easily. In particular, when the expression vector is constructed so that the protein of the present invention is expressed in a form of a "fusion protein" comprising the protein of the present invention linked to other protein (for example, a labeled peptide), the protein of the present invention can be isolated and purified easily.

Examples of such "other protein" include various peptides such as signal peptides (i.e. peptides consisting of 15 to 30 amino acid residues which present at N-terminals of many proteins and function inside cells for the selection of proteins in the intracellular membrane penetration mechanism: for example, OmpA, OmpT, Dsb and so on), protein kinase A, protein A (a protein having a molecular weight of about 42,000, which is a constituent of a cell wall of *Staphylococcus aureus),* glutathione S-transferase, His tag (a sequence of 6 to 10 histidine residues arranged in a row), myc tag (a sequence of 13 amino acids from cMyc protein), FLAG peptide (a marker for analysis consisting of 8 amino acid residues), T7 tag (consisting of first 11 amino acid residues of gene 10 protein), S tag (consisting of 15 amino acid residues from pancreatic RNase A), HSV tag, pelB (a sequence consisting of 22 amino acids of *Escherichia coli* outer membrane protein pelB), HA tag (consisting of 10 amino acid residues from haemagglutinin), Trx tag (thioredoxin sequence), CBP tag (calmodulin binding peptide), CBD tag (cellulose binding domain), CBR tag (collagen binding domain), β-lac/blu (β-lactamase), β-gal (β-galactosidase), luc (luciferase), HP-Thio (His-patch thioredoxin), HSP (heat shock peptide), Lnγ (laminin-γ-peptide), Fn (fibronectin partial peptide), GFP (Green fluorescent peptide), YFP (yellow fluorescent peptide), CFP (cyan fluorescent peptide), BFP (blue fluorescent peptide), DsRed and DsRed2 (red fluorescent peptides), MBP (maltose binding peptide), LacZ (lactose operator), IgG (immunoglobulin G), avidin, and protein G.

Expression vectors into which the protein of the present invention has been incorporated are extracted and purified and then introduced into host cells. Host cells can be selected as appropriate depending on the kind of the expression vector to be used and are not particularly limited. Host cells may be either eukaryotic cells (e.g., mammalian cells, yeast, insect cells, and so on) or prokaryotic cells (e.g., *Escherichia coli, Bacillus subtilis,* and so on).

For example, when an expression vector for mammalian cells is used, mammalian cells can be used as host cells. The kind of the mammalian cells referred to herein is not particularly limited and can be selected as appropriate in terms of the object, expression efficiency, or the like. For example, chinese hamster ovary derived cell line (i.e., CHO cell line) and so on may be used.

The expression vector into which the polynucleotide (DNA) of the present invention has been incorporated is introduced into such cells and cultivated by conventional methods. The conditions of culture may be selected as appropriate depending on the kind of vector/host cells, objects, and so on.

In this manner, by culturing the cells into which the polynucleotide of the present invention has been introduced and collecting the culture product, the protein of the present invention is produced. As appropriate, the protein of the present invention may be further purified by using known extraction and purification methods.

### EXAMPLES

Hereinafter, the present invention is described in more detail by referring to examples in which SAT-I (a kind of N-linked glycoprotein) is used.

### <1> Materials, methods, and so on

The materials, methods, and so on used in the present invention are as follows.
- LIPOFECTAMINE^{™} 2000 Reagent: manufactured by Life Technology, Inc..
- NUTRIENT MIXTURE F-12 HAM (HAM): manufactured by SIGMA.
- PVDF membrane (i.e., Immobilon): manufactured by MILLIPORE.
- X-ray film (i.e., Medical X-ray film): manufactured by Kodak.
- Endo H: manufactured by New England Biolabs.
- PNGase F: manufactured by New England Biolabs.
- Tunicamycin: manufactured by SIGMA.
- Kifnecin: manufactured by CALBIOCHEM.
- Castanospermine: manufactured by CALBIOCHEM.
- CMP-sialic acid: manufactured by SIGMA.
- LacCer: manufactured by Matreya, Inc.
- SepPak Plus 18: manufactured by Waters Associates (Milford, MA)).
- Silica gel HPTLC: manufactured by Merck.
- Anti-SAT-I antibody: anti-SAT-I rabbit IgG antibody (i.e., a polyclonal antibody prepared by using 51 residues at C-terminal side of mSAT-I as an antigen).
- 5×SDS sample buffer: 62.5 mM Tris (pH 6.8), 2% SDS, 10% glycerol, 0.001% BPB, and 5% 2-mercaptoethanol.
- Running buffer: 25 mM Tris (pH 8.9), 192 mM glycine, 0.1% SDS.
- Transfer buffer: 25 mM Tris, 192 mM glycine, 20% methanol.
- TBS-T buffer: 137 mM NaCl, 20 mM Tris (pH 7.5), 0.05% Tween 20.
- Blocking buffer: 5% skim milk solution prepared by using TBS-T buffer.
- Cell suspension solution: 5% glycerol, 15 mM sodium cacodylate, 0.1% Lubrol (manufactured by ICN Biochemicals Inc.), protease inhibitor mixture (CompleteTM EDTA free): manufactured by Roche Applied Science.
- 4×Reaction mixture: 400 mM sodium cacodylate, 40 mM MgCl₂, 1.6% Triton X-100.
- PBS: 137 mM NaCl, 2.68 mM KCl, 10 mM Na₂HPO₄,1.76 mM KH₂PO₄.
- IP buffer: 50 mM Tris (pH 7.4),150 mM NaCl, 2 mM NaF, 1 mM EDTA, 1 mM EGTA, 1% Triton X-100, 1 mM PMSF, protease inhibitor mixture (CompleteTM EDTA free): manufactured by Roche Applied Science.
- Cell culture: CHO cells were cultured by using NUTRIENT MIXTURE F-12 HAM medium containing 10% (v/v) FBS, 100 units/ml penicillin, 100 ng/ml streptomycin, 9.4% (v/v) sodium hydrogen carbonate, and 100 mM L-glutamine under conditions of 37°C and 5% CO₂. The cells were recovered by using 0.25% trypsin/1 mM EDTA solution by subculturing once for every 3 days.
- Introduction of SAT-I gene into CHO cells
CHO cells were inoculated on a 6-well plate and cultivated until 90% to 95% confluent was achieved. Then, the cells were gently washed with a medium that contained neither antibiotics nor serum (i.e., HAM (-/-)), followed by addition of 2.25 ml of the same medium and 250 µl of FBS.

Then, a plasmid (2 µg; pcDNA3.1 Zeo(+) (Invitrogen) into which the mSAT-I gene was incorporated) and 10 µl of LIPOFECTAMINE^{™} 200 Reagent were mixed with 250 µl of HAM (-/-), respectively, and the resultants were left to stand at room temperature for 5 minutes. After that, the both were mixed with each other and the resultant was left to stand at room temperature for 20 minutes. Then the resultant was added to the cells, followed by culturing for 24 hours. In the case where an inhibitor such as tunicamycin was added, the medium was replaced 6 hours after the transfection and inhibitors were added thereto (final concentration: tunicamycin: 2.5 µg/ml, Kifnecin: 5 µg/ml, castanospermine: 1 mM) and the mixture was cultured for 18 hours.

### • SDS-PAGE

5% polyacrylamide gel as a concentration gel and 7.5% or 10% polyacrylamide gel as a separation gel were used. 5×SDS sample buffer was added in a volume 1/3 of that of the sample solution and the mixture was boiled at 37°C for about 3 minutes to denature the sample to prepare a sample for electrophoresis. By using a mini-real slab gel electrophoresis apparatus (manufactured by Biocraft, Inc.), electrophoresis was performed at a constant current of 20 mA for about 80 minutes. As a molecular weight marker serving as an index for molecular weight, Prestained Protein Marker was used. The molecular weights of the proteins included in the molecular weight marker were 175, 83, 62, 47.5, 32.5, 25, 16.5, and 6.5 kDa.

### • Immunoblotting

After SDS-PAGE was completed, the gel and the activated PVDF membrane were each equilibrated with transfer buffer by shaking for about 5 minutes. By using a semi-dry type transfer apparatus (trade name: Trans-Blot SD, manufactured by Biorad, Inc.), the protein in the gel was transferred onto the PVDF membrane at 10 V for 30 minutes. The PVDF membrane was blocked by using a blocking buffer at room temperature for 1 hour. After that, a primary antibody (i.e., anti-SAT-I antibody) was diluted 1,000 fold with the blocking buffer. The PVDF membrane was dipped in the resultant solution and shaken at overnight 4°C . After that, the PVDF membrane was dipped in a secondary antibody solution (i.e., anti-rabbit IgG labeled with HRP) diluted about 5,000 folds with TBS-T buffer, and allowed to react at room temperature for 1 hour.

The PVDF membrane after the reaction was colored with a Western blotting detecting reagent (i.e., ECL or ECL Plus (manufactured by Amersham Biosciences) or Lumi-Light Plus (manufactured by Roche Diagnostics Co., Ltd.). For detection, an X-ray film was used.

### • Sugar chain cleaving reaction

Endo H or PNGase F was added to samples prepared by the alkali-acetone method and the samples were allowed to react at 37°C for 1 hour. Then, the samples were analyzed by immunoblotting.

### • Assay of SAT-I activity

CHO cells into which the mSAT-I gene was introduced (placed on a 6-well plate) were washed with ice-cold PBS and then 80 µl of solution for cell suspension was added to the cells and the cells were collected. After the cells were sonicated (once for 10 seconds), the sonicated product was centrifuged at 4°C and 15,000×g for 10 minutes to collect the supernatant, which was used as an enzyme source. Further, 10.68 µl of 1 mg/ml LacCer was dried at room temperature, to which 10 µl of 4×Reaction mixture was added. The resultant was sonicated to suspend LacCer. To the suspension were added 20 µl of the enzyme source and a 1:4 mixture of radiolabeled CMP-sialic acid (i.e., Cytidine 5'-monophosphate, [4,5,6,7,8,9-¹⁴C]sialic acid (manufactured by NEN Life Science Products)) and non-radiolabeled CMP-sialic acid were added and the resultant mixture was allowed to react at 37°C for 2 hours.

A fraction that contained lipids was purified from the solution after the reaction by using Sep-Pak Plus 18 and the resultant was applied to an HPTLC plate. Lipids were separated with a developing solvent (chloroform/methanol/0.2% CaCl₂ (55/45/10)).

### • Creation of SAT-I mutant

Various mutants of mSAT-I were created by using mSAT-I as a template and QuickChange site-directed mutagenesis kit (manufactured by Stratagene) according to the recommendations described in the manual attached to the kit. The primers used for creating the various mutants are shown below.

N180Q:
5'-CTCTGAACACGTTGGGCAGAAAACTACTATAAGG-3' (SEQ ID NO: 19)
5'-CCTTATAGTAGTTTTCTGCCCAACGTGTTCAGAG-3' (SEQ ID NO: 20)
N180K:
5'-CTCTGAACACGTTGGGAAGAAAACTACTATAAGG-3' (SEQ ID NO: 21)
5'-CCTTATAGTAGTTTTCTTCCCAACGTGTTCAGAG-3' (SEQ ID NO: 22)
N180S:
5'-CTCTGAACACGTTGGGAGCAAAACTACTATAAGG-3' (SEQ ID NO: 23)
5'-CCTTATAGTAGTTTTGCTCCCAACGTGTTCAGAG-3' (SEQ ID NO: 24)
H177D, N180S:
5'-GAGGGTTACTCTGAAGAGGTTGGGCAGAAAACTACTATAAGG-3' (SEQ ID NO: 25)
5'-CCTTATAGTAGTTTTGCTCCCAACGTCTTCAGAGTAACCCTC-3' (SEQ ID NO: 26)
H177D:
5'-GAGGGTTACTCTGAAGAGGTTGGGCAGAAAACTAC-3' (SEQ ID NO: 27)
5'-GTAGTTTTATTCCCAACGTCTTCAGAGTAACCCTC-3' (SEQ ID NO: 28)
N224Q:
5'-GCAATGGTAAAACAGGAAAGCCTGCCC-3' (SEQ ID NO: 29)
5'-GGGCAGGCTTTCCTGTTTTACCATTGC-3' (SEQ ID NO: 30)
N224K:
5'-GCAATGGTAAAAAAGGAAAGCCTGCCC-3' (SEQ ID NO: 31)
5'-GGGCAGGCTTTCCTTTTTTACCATTGC-3' (SEQ ID NO: 32)
N224D:
5'-GCTTCAAGCAATGGTAAAACAGGAAAGCCTGCCCTTTTG-3' (SEQ ID NO: 33)
5'-CAAAAGGGCAGGCTTTCATCTTTTACCATTGCTTGAAGC-3' (SEQ ID NO: 34)
N334Q:
5'-CTGGCAGGTCATGCACCAGGTGACCACAGAGACCAAG-3' (SEQ ID NO: 35)
5'-CTTGGTCTCTGTGGTCACCTGGTGCATGACCTGCCAG-3' (SEQ ID NO: 36)
N334K:
5'-CTGGCAGGTCATGCACAAGGTGACCACAGAGACCAAG-3' (SEQ ID NO: 37)
5'-CTTGGTCTCTGTGGTCACCTTGTGCATGACCTGCCAG-3' (SEQ ID NO: 38)
T336Q:
5'-CAGGTCATGCACAAGGTGACCACAGAGACCAAGTTCCTC-3' (SEQ ID NO: 39)
5'-GAGGAACTTGGTCTCTGTCTGCACATTGTGCATGACCTG-3' (SEQ ID NO: 40)

### • Trypsin sensitivity test

After washing of CHO cells into which the mSAT-I gene was transfected (placed on a 6-well plate) with ice-cold PBS, 100 µl of IP buffer was added thereto and the cells were collected. The cells were sonicated (once for 10 seconds), and centrifuged at 4°C and 15,000×g for 10 minutes. The supernatant was collected, to which trypsin was added such that the final concentration was 0, 10, 20, 50, or 100 ng/µl and the resultants were allowed to react at 37°C for 30mintues. After that, 10% SDS was added to a final concentration of 1% to stop the reaction.

### <2> Results

### (1) Interspecies comparison of N-glycosylation sites

Cloned SAT-Is of human, mouse, rat, and zebra-fish each have a transmembrane region in the N-terminal side and have sequences that are conserved in sialic acid transferase, called "sialyl motif L", "sialyl motif S", and "sialyl motif VS". The sialyl motif L is known to participate in binding to CMP-sialic acid, while functions of the other motifs have not been defined yet. The results of amino acid sequence comparison between hSAT-I (SEQ ID NO: 42), mSAT-I (SEQ ID NO: 4) and zSAT-I (SEQ ID NO: 41) are shown in Table 1.

Further, N-linked sugar chains are known to link to the Asn residue of the amino acid sequence referred to as "Asn-Xaa-Thr/Ser" present in a glycoprotein.

In Fig. 1, the dotted crossbar line portion indicates a transmembrane region, the first solid crossbar line portion indicates a sialyl motif L, the second solid crossbar line portion indicates a sialyl motif S, and the third solid crossbar line portion indicates a sialyl motif VS. Further, the portion boxed with a bold line indicates a portion to which an N-linked sugar chain can be linked (N-glycosylation site). It has been shown that N-linked sugar chains can be linked to N180, N224, and N334 of mSAT-I.

Comparison of the amino acid sequences at the N-glycolation site indicates that except for the site in the sialyl motif L, the amino acid sequences at the N-glycosylation site are not conserved in the species. This suggests two possibilities that the sugar chains other than the sialyl motif L are not important to SAT-I, or that the modified amino acid sequence alternates the function of the sugar chain.
The comparison of the amino acid sequence of mSAT-I with the amino acid sequences of cloned SAT-Is from bovine, dog, rat, chicken, medaka, and a tetradon was performed according to the procedures of Clustal W (Thompson, J.D., Higgins, D.G, and Gibson, T.J. (1994) Nucleic Acids Res. 22,4673-4680), and BOXSHADE (Institute for Animal Health, Surrey, UK), and indicated that regarding the amino acid sequence portions of the bovine, dog, rat, and chicken corresponding to the amino acid sequences of the N-glycosylation sites (around N 180, N224, and N334) of mSAT-I, percentages of amino acid conservation were high. On the other hand, in medaka and tetradon, percentages of amino acid conservation were relatively low and the amino acids corresponding to N180, N224, and N334 were different, that is, they were S, K, and D, respectively.
Similarly, comparison of the amino acid sequence of the human-derived sialyltransferase with those of various enzyme families indicated that differences were observed among the enzyme families, that, for example, N at the 180th position in the glycosylation site present in the sialyl motif L in SAT-I was S or T, and H at the 177th position was D.
Further, the glycosylation site and its number in various sialyltransferases are known to differ from each other among species or enzyme families; this fact is considered to reflect a change in functional regulation of glycosyltransferases in the process of evolution to higher organisms. That is, the difference suggests the possibility that substitution of a particular amino acid allows to create an enzyme that retains its activity without sugar chains.

### (2) Study on the presence or absence of sugar chains linked to SAT-I and kinds thereof

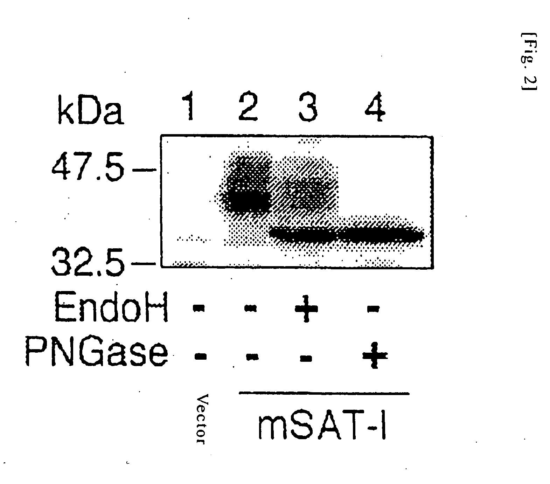

An anti-SAT-I antibody (polyclonal antibody) was prepared by using 51 residues at C-terminal of mSAT-I as an antigen. mSAT-I was transiently expressed by using CHO cells, and a lysate of the cells was subjected to SDS-PAGE and then to immunoblotting with an anti-SAT-I antibody. A lysate of the cells treated with Endo H or PNGase F was also subjected to immunoblotting with an anti-SAT-I antibody. The results are shown in Fig. 2.

The molecular weight of mSAT-I estimated by the amino acid sequence of mSAT-I is about 40 kDa. However, a major band having a larger molecular weight than that of mSAT-I was detected near 42 kDa and a broad band was detected from 45 kDa to 48 kDa (Lane 2 in Fig. 2). The result suggested that some modification was made to mSAT-I.
Further, treatment with PNGase F resulted in all the mSAT-I bands aggregated to a molecular weight of 40 kDa (Lane 4 in Fig. 2), while treatment with Endo H did not lead to complete cleavage of sugar chains, so that an Endo H-resistant band was detected (Lane 3 in Fig. 2). These results indicate that a complex sugar chain modified by the Golgi apparatus is linked to MAT-I.

### (3) Influence of sugar chain on SAT-I activity

When mSAT-I was transiently expressed by using CHO cells, the cells were treated with an inhibitor (tunicamycin, Kifnecin, or castanospermine) of sugar chain processing in ER. Treatment with tunicamycin inhibited dolicholpyrophosphate-N-acetylglucosamine formation to suppress the glycosyltransfer to the protein. Treatment with Kifnecin inhibited mannosidase, resulting in generation of mSAT-I having a high mannose type sugar chain immediately before the transportation to the Golgi apparatus, preventing the formation of mSAT-I having a complex type sugar chain. Treatment with castanospermine causes inhibition of glucosidases I and II to suppress the interaction with calnexin or calreticulin that interacts by recognizing monoglucosylated sugar chains.

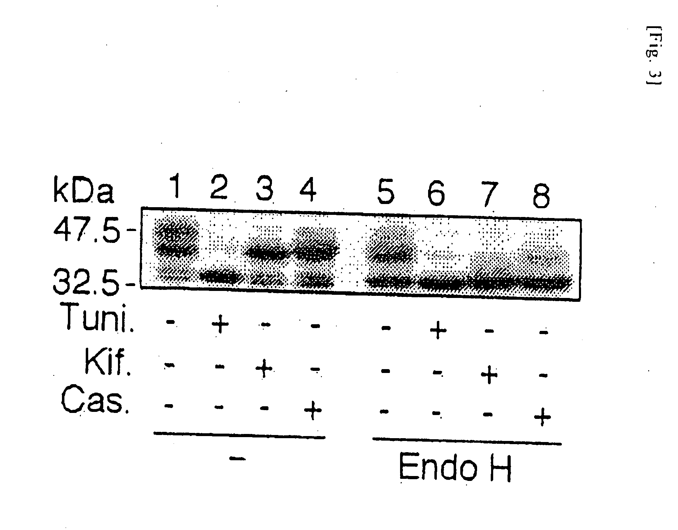

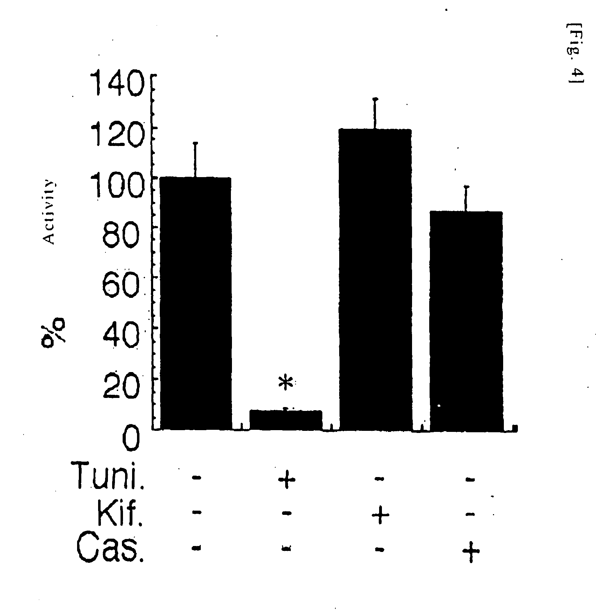

Lysates of the cells treated with those inhibitors were prepared in the presence or absence of Endo H and the lysates were subjected to SDS-PAGE, followed by immunoblotting with an anti-SAT-I antibody. Results are shown in Fig. 3. Results of assay of SAT-I activity by using the lysates are shown in Fig. 4.

Fig. 3 indicates that treatment with tunicamycin completely suppressed the glycosyltransfer to SAT-I and that treatment with Kifnecin or castanospermine resulted in linking of only high mannose type sugar chain (cleaved by Endo H).

Fig. 4 indicates that the activity of SAT-I to which no sugar chain was added by the treatment with tunicamycin decreased remarkably. On the other hand, SAT-I that retained the high mannose type sugar chain obtained by the treatment with Kifnecin or castanospermine had an activity equivalent to that of mSAT-I subjected to treatment without the inhibitors. These results indicated that a high mannose type sugar chain is essential for the activity of SAT-I and interaction with calnexin or calreticulin is unnecessary in the process of usual SAT-I folding.

(4) Creation of "N 180Q", "N224Q", "N334Q", and "N 180Q, N224Q, N334Q" mutants Mutants (i.e., "N180Q", "N224Q", "N334Q", and "N180Q, N224Q, N334Q") in which any one or all of Asns in three N-glycosylation sites (i.e., N180, N224, and N334) present in mSAT-I was substituted by Gln were created.

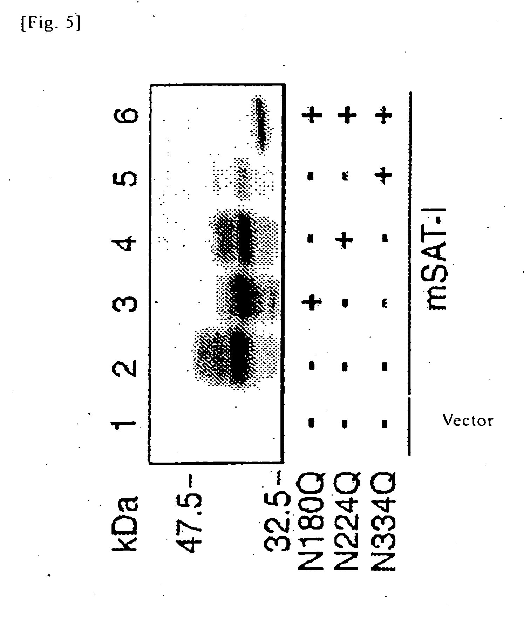

These mutants were transiently expressed in CHO cells and the cell lysates were subjected to SDS-PAGE and immunoblotting with an anti-SAT-I antibody was performed. Results are shown in Fig. 5.

As a result, a decrease in molecular weight was observed in all the mutant and the "N180Q, N224Q, N334Q" mutant had the same molecular weight as that of the wild type mSAT-I treated with PNGase.

Further, in the N334Q mutant and the."N180Q, N224Q, N334Q" mutant, the amount of SAT-I remarkably decreased. The above-mentioned results indicated that a sugar chain was added to all the three N-glycosylation sites in mSAT-I and no modification of sugar chain occurred in other sites.

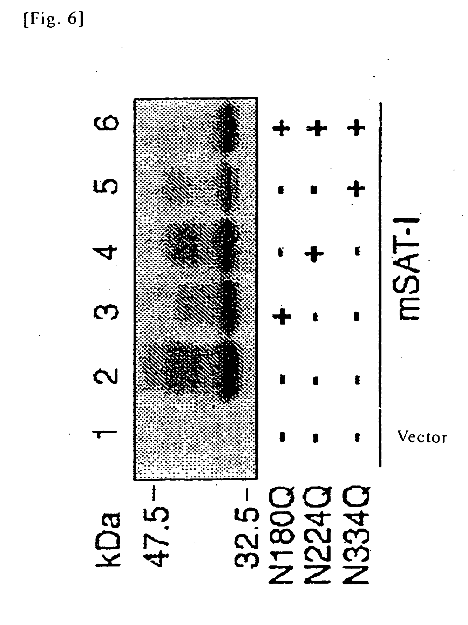

Then, the lysates of the cells in which various mutants were respectively expressed were treated with Endo H (37°C, 1 hour), and the treated lysates were subjected to SDS-PAGE, followed by immunoblotting with an anti-SAT-I antibody. Results are shown in Fig. 6.

As a result, Endo H-resistant band specifically decreased in the N180Q mutant. The decrease in Endo H-resistant band in the N334Q mutant was thought to be non-specific thereto from the fact that the Endo H-sensitive band similarly decreased.

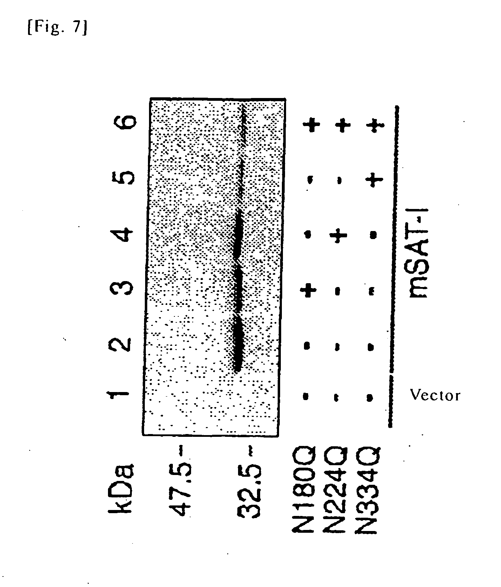

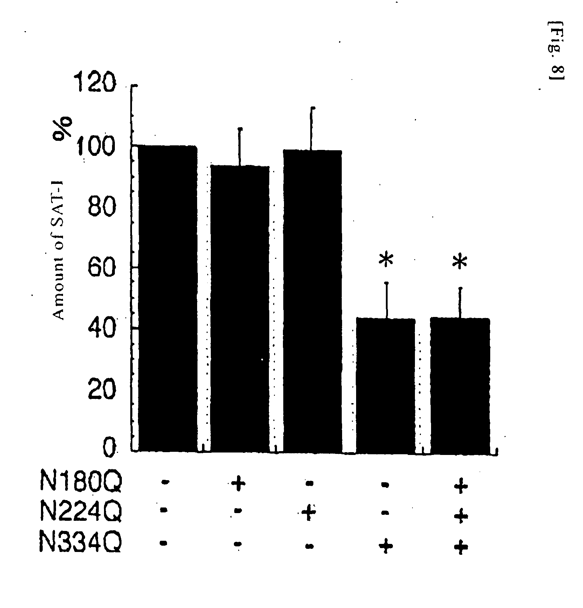

Then, the lysates of the cells in which various mutants were respectively expressed were treated with PNGase F (37°C, 1 hour), and the treated lysates were subjected to SDS-PAGE, followed by immunoblotting with an anti-SAT-I antibody, to compare the amount of total SAT-I. Results of immunoblotting are shown in Fig. 7 and results of numerical conversion of the amount (mean value of three immunoblotting results) based on the results in Fig. 7 are shown in Fig. 8.

As a result, in the mutant N334Q and the mutant "N180Q, N224Q, N334Q", a decrease in amount of protein of about 50% was indicated as compared with the wild type. However, when the cells were treated with tunicamycin, no such decrease was found, and it is unlikely that the sugar chain participates in the decrease in an amount of SAT-I. Further, results of a pulse label experiment with [³⁵S]-methionine indicated that the decrease occurred at the level of translation of protein. Therefore, it is considered that since the secondary structure of the mRNA was remarkably changed by mutation, the translation efficiency to a protein decreased. As a result, the amount of the proteins of the mutant N334Q and the mutant "N 180Q, N224Q, N334Q" decreased.

### (5) Comparison of trypsin sensitivities among various mutants

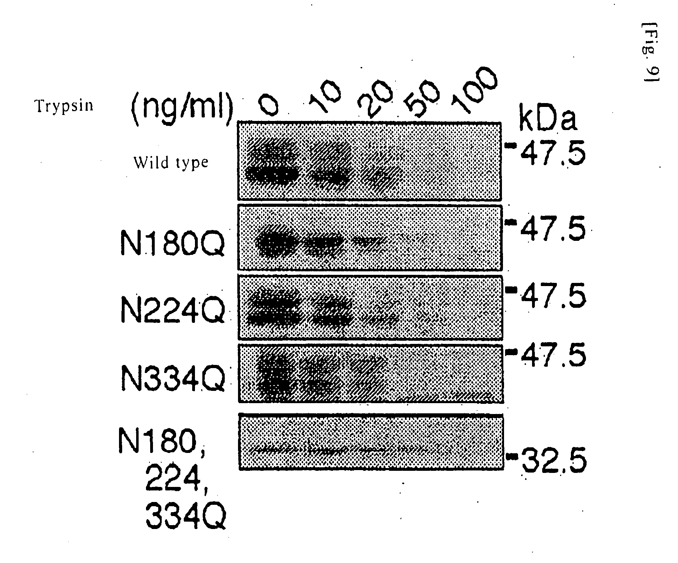

An increase in sensitivity to proteases such as trypsin due to a miss folding of a protein has been known. Therefore, whether the structures of the mutants "N180Q", "N224Q", "N334Q", and "N180Q, N224Q, N334Q" were significantly changed was examined by comparing sensitivities of the mutants to trypsin. That is, mSAT-I was transiently expressed by using CHO cells, and a lysate of the cells was prepared. The lysate were treated with 0, 10, 20, 50, or 100 ng/ml trypsin at 37°C for 1 hour. Results are shown in Fig. 9.

As shown in Fig. 9, no significant difference was observed in the sensitivity to trypsin between the wild type and the mutants. Therefore, it was suggested that the possibility that lack of sugar chains would remarkably change the conformation of SAT-I was low.

### (6) Influence of sugar chain on mSAT-I activity

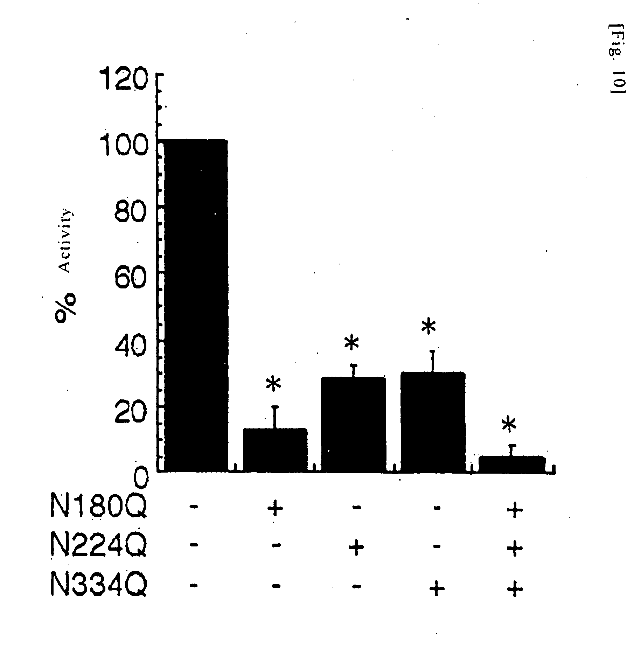

Each mutant was transiently expressed by using CHO cells and the cell lysates were used as an enzyme source and their enzyme activities were assayed. Results are shown in Fig. 10.

As a result, in all the mutants N180Q, N224Q, N334Q, and "N180Q, N224Q, N334Q", the activities decreased remarkably as compared with the wild type. In particular, the mutant N180Q had an activity lower than those of the mutants N224Q and N334Q, and in the mutant "N180Q, N224Q, N334Q" in which no sugar chain was linked, almost all the enzyme activity was lost. The above-mentioned results indicated that the presence of each sugar chain was essential for the enzyme activity of mSAT-I.

### (7) Retention of function of N-linked sugar chains by amino acid substitution (retention of physiological activity of protein)

Whether the function of an N-linked sugar chain is retained by substituting an amino acid at a particular portion (whether the physiological activity of the protein is retained without N-linked sugar chains modification) was examined by using mSAT-I.

First, sugar chains which link to N224 were examined. Mutants N224K (SEQ ID NO: 6) and N224D were created and their activities were compared with those of the wild type and the mutant N224Q.

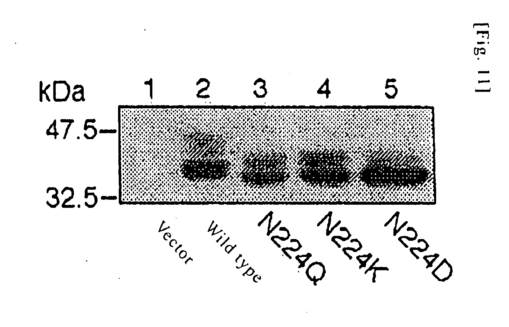

These mutants were transiently expressed by using CHO cells and the cell lysates were subjected to SDS-PAGE, and immunoblotting with an anti-SAT-I antibody was performed. Results are shown in Fig. 11. The results indicated that the mutants N224K and N224D showed band patterns similar to that of the mutant N224Q.

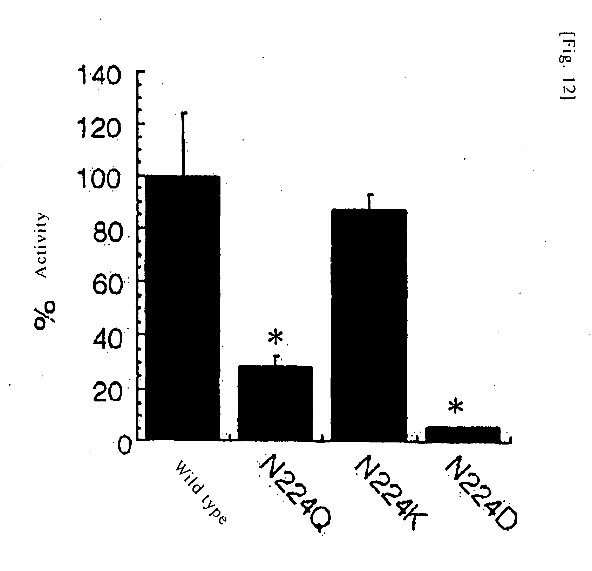

The results of assay of the SAT-I activity of each mutant are shown in Fig. 12. The results indicated that the mutant N224K showed an activity of the same level as that of the wild type. On the other hand, in the mutant N224D in which substitution was made by D (Asp) having an opposite charge to K (Lys), the activity decreased more than that of the mutant N224Q. These results indicated that the function of the sugar chain which links to N224 was retained by substituting N224 by K (Lys) having a positive charge. The results also indicated that by performing such a substitution of an amino acid, the physiological activity (the enzymatic activity of SAT-I) of the protein was retained without N-linked sugar chain modification.

Then, the sugar chain which links to N334 was examined. The mutants N334K and T336Q (SEQ ID NO: 8) were created and their activities were compared with those of the wild type and a mutant N334Q.

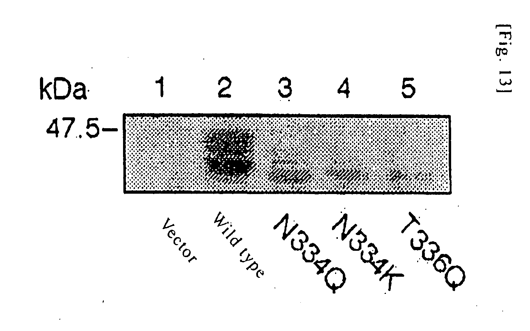

These mutants were transiently expressed by using CHO cells and the cell lysates were subjected to SDS-PAGE, and immunoblotting with an anti-SAT-I antibody was performed. Results are shown in Fig. 13. The results indicated that both the mutants N334K and T336Q showed band patterns similar to that of the mutant N334Q.

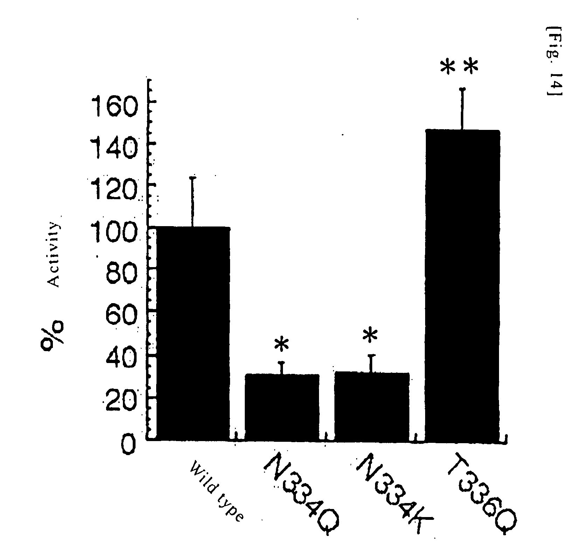

Further, results of assay of SAT-I activity of each mutant are shown in Fig. 14. The results indicated that the mutant N334K only showed an activity of the same level as that of the mutant N334Q while the mutant T336Q showed an activity about 1.5 folds higher than that of the wild type. These results indicated that the function of the sugar chain which links to N334 was retained by substituting T336 by Q (Gln). The results also indicated that by performing such a substitution of an amino acid, the physiological activity (the enzymatic activity of SAT-I) of the protein was retained without N-linked sugar chain modification.

Then, the sugar chain which links to N180 was examined. Mutants N180K, N180S (SEQ ID NO: 10), "H 177D, N 180S" (SEQ ID NO: 12), and H 177D (SEQ ID NO: 14) were created and their activities were compared with those of the wild type and a mutant N180Q.

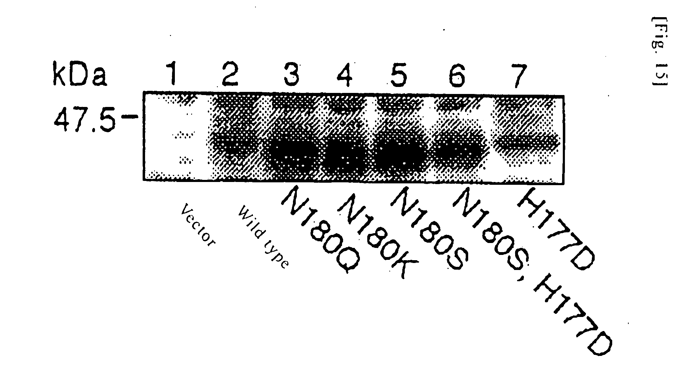

These mutants were transiently expressed by using CHO cells and the cell lysates were subjected to SDS-PAGE, and immunoblotting with an anti-SAT-I antibody was performed. Results are shown in Fig. 15. The results indicated that the mutants N180S and "H 177D, N 180S" had a major band around 42 kDa similar to that of the mutant N 180Q and the mutant N180K had major bands at around 42 kDa and 40 kDa. The mutant H177D showed a similar band pattern to that of the wild type.

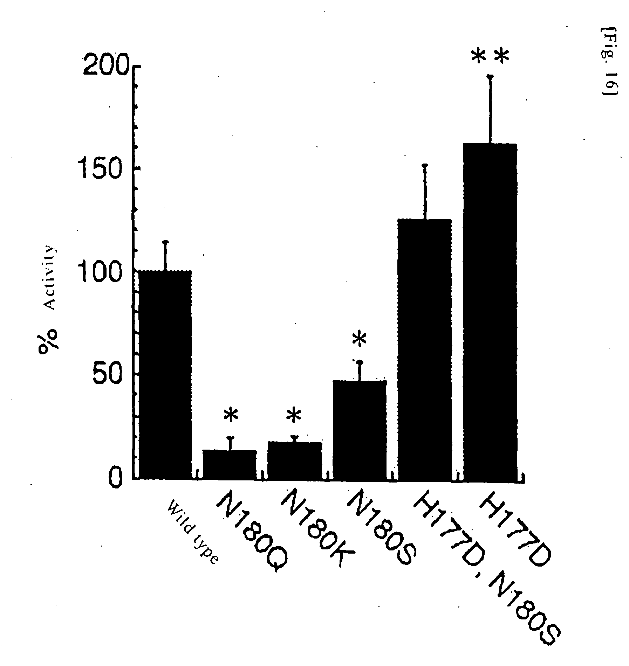

Further, results of assay of SAT-I activity of each mutant are shown in Fig. 16. The results indicated that the mutant N180K only showed an activity of the same level as that of the mutant N 180Q while the mutant N180S showed an activity of about 50% of that of the wild type, and the mutant "H177D, N180S" had an activity equivalent to or higher than that of the wild type. Further, the mutant H177D showed an activity of about 1.5 folds higher than that of the wild type. These results indicated that the function of the sugar chain which links to N180 was retained by substituting H177 by D (Asp) and/or substituting N180 by S (Ser). The results also indicated that by performing such a substitution of an amino acid, the physiological activity (the enzymatic activity of SAT-I) of the protein was retained without N-linked sugar chain modification.

Then, the mutant "N180S, N224K, T336Q" having no sugar chain modification site and the mutant "H177D, N180S, N224K, T336Q" (SEQ ID No: 16) were created and their activities were compared with those of the wild type and the mutant "N180Q, N224Q, N334Q".

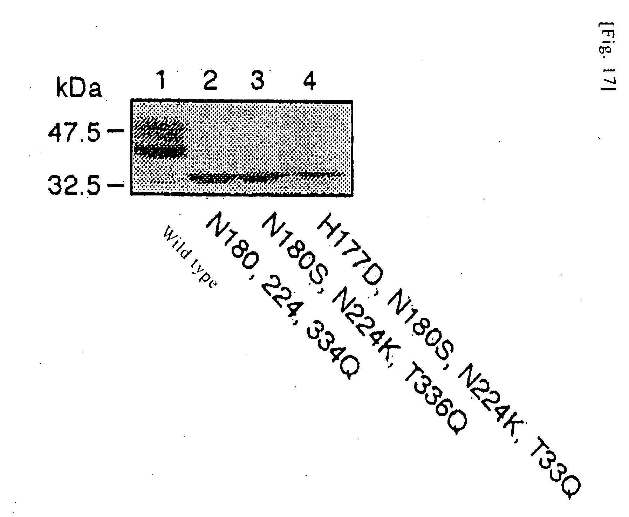

These mutants were transiently expressed by using CHO cells and the cell lysates were subjected to SDS-PAGE, and immunoblotting with an anti-SAT-I antibody was performed. Results are shown in Fig. 17. The results indicated that both mutants showed a band around 35 kDa in a manner similar to the case of PNGase F treatment.

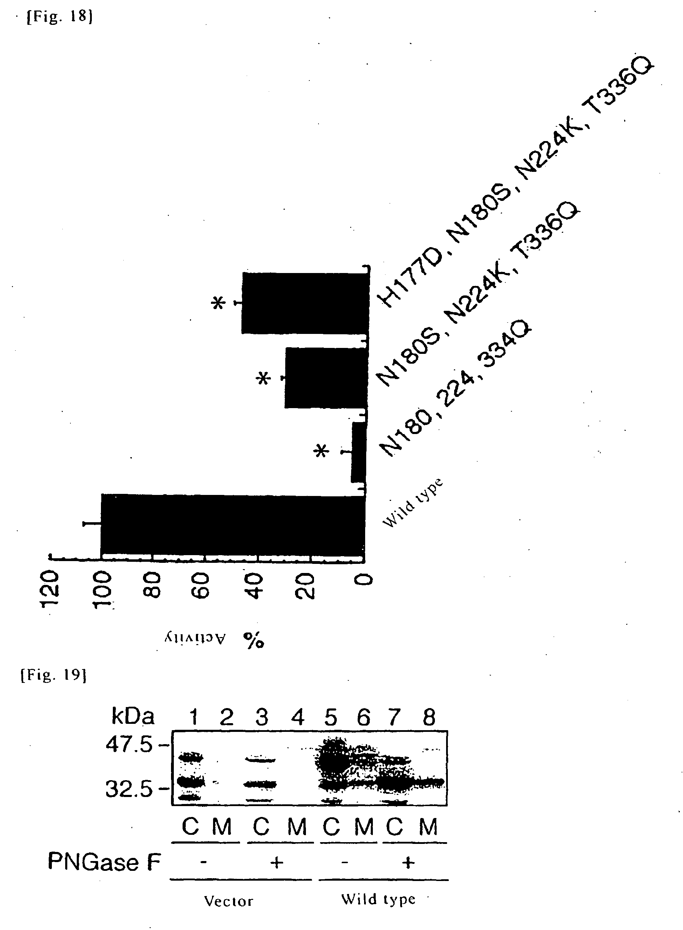

Further, results of assay of SAT-I activity of each mutant are shown in Fig. 18. The results indicated that the mutant "N180S, N224K, T336Q" showed an activity of about 40% of that of the wild type, and the mutant "H177D, N180S, N224K, T336Q" showed an activity of about 50% of that of the wild type. Further, separately, the mutant "N224K, T336Q" (SEQ ID NO: 18) was created and the activity of SAT-I was assayed similarly. As a result, the mutant showed an activity equivalent to that of the wild type.

The results indicated that, in the amino acid sequence of N-linked glycoprotein having the amino acid sequence motif (I) and/or (II) described below, substitution of an amino acid in the amino acid sequence motif (I) and/or (II) by other amino acid(s) according to one or more of rules (A) to (C) described below allowed the physiological activity of the glycoprotein before the mutation is introduced to be retained without being subjected to modification by an N-linked sugar chain in the following amino acid sequence containing the amino acid to be substituted:
(I) Asn Xa1 Xa2 (SEQ ID NO: 1); and
(II) Xa3 Val Gly Asn Xa1 Xa2 (SEQ ID NO: 2)
(in the amino acid sequence motif (I) and (II), Xa1 indicates an amino acid other than Pro, Xa2 indicates Thr or Ser, and Xa3 indicates His or Asp);
(A) substitution of Asn by Lys or Ser;
(B) substitution of Xa2 by Gln; and
(C) substitution of Xa3 by Asp.

### (8) Influence of amino acid substitution on extracellular secretion

Experiments on the extracellular secretion of SAT-I were performed.
In a similar manner as the transfection using the above-described CHO cells, HEK293 cells were cultured in a Dulbecco modified Eagle medium and SAT-I was transiently expressed in the HEK293 cells by using a pcDNA3 vector having a wild type mSAT-I inserted therein (wild type pcDNA3.1 Zeo(+)-mST3Gal-V). After the culture, the cell lysates and the culture were separately collected and the lysate of the cell lysates and the culture were subjected to SDS-PAGE, and immunoblotting with an anti-SAT-I antibody was performed. Further, each of the lysate of the cells and the medium was treated with PNGase F and then immunoblotting was similarly performed, and the results thereof were compared (Fig. 19). As a control, the pcDNA3 vector was used.
SAT-I secreted in the medium showed broad bands of 45 kDa and 38 kDa. It was predicted that the 45 kDa band retains an N-linked sugar chain since the 45 kDa band was not detected in immunoblotting after the PNGase F treatment. The 45 kDa band was resistant to Endo H.

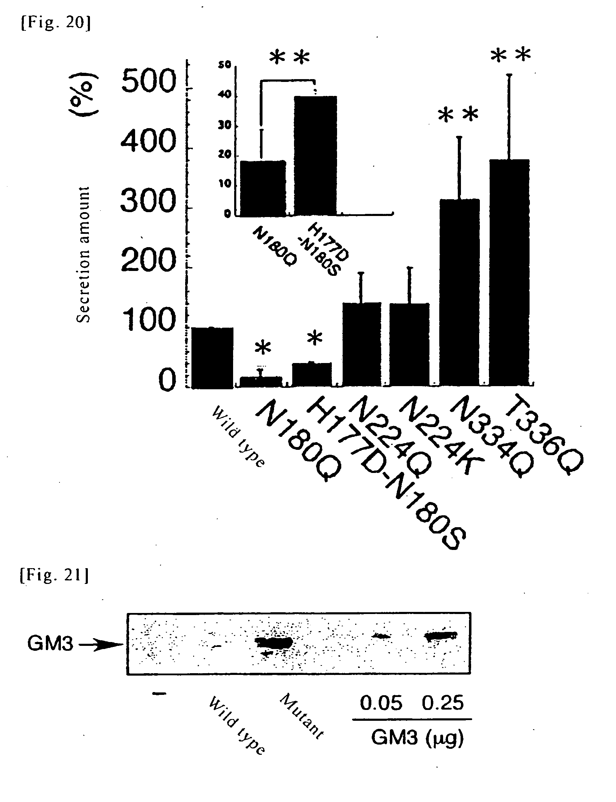

Further, by using the HEK293 cells, various mutants of mSAT-I created as described above (the mutants N 180Q, "H177D, N180S", "N224Q", N224K, N334Q, and T336Q) and the wild type mSAT-I were transiently expressed, and mSAT-I extracted from the cell lysates and mSAT-I secreted in the culture were subjected to PNGase F treatment and then subjected to SDS-PAGE, and immunoblotting with an anti-SAT-I antibody was performed. The bands were analyzed by measuring concentrations thereof, and a ratio of the amount of protein secreted to the substrate to the amount of protein present in the cells was calculated. Further, setting the ratio of the wild type mSAT-I as 100%, the secretion amount of each mutant was calculated (Fig. 20).
The secretion amounts of the mutant N224Q and of the mutant N224K were substantially the same as that of the wild type. On the other hand, the mutant N 180Q had a secretion amount of 20% of that of the wild type and the mutant "H177D, N180S" had a secretion amount of 50% of the wild type. Surprisingly, the mutant N334Q and the mutant T336Q had a secretion amount higher than 300% of the wild type. These results revealed that the N-linked sugar chain linked to the N at the 180th position in SAT-I played an important role in secretion into a medium and on the other hand, the N-linked sugar chain linked to the N at the 334th position participates in the function of retaining the protein in the cells.
The secretion amount of the mutant "H177D, N180S" which retained an activity substantially equivalent to that of the wild type was approximately two times the secretion amount of the mutant N 180Q which showed an activity of only about 20% of the activity of the wild type.
The mutant N 180Q had an abnormal structure that is enzymatically inactive due to loss of the N-linked sugar chain, so it had an activity of about 20% of the wild type and a secretion amount of about 20% of the wild type. On the other hand, the mutant "H177D, N180S" had an enzyme activity substantially equivalent to that of the wild type, so it was suggested that it retained the enzyme activity structure. It was predicted that the decrease in the secretion amount was due to the loss of the N-linked sugar chain linked to the N at the 180th position.
These results suggested that not only the N-linked sugar chain linked to N at the 180th position but also the protein structure near this site is involved in the activity and secretion of SAT-I.

When the protein of the present invention is produced by genetic engineering, regulation of the function relating to the extracellular secretion is important. The method of obtaining the target protein of the present invention from the culture medium is more convenient and efficient than the method of obtaining the target protein of the present invention from cultured cells.

### (9) Expression of mutant "H177D, N180S, N224K, T336Q" of mSAT-I in Escherichia coli

### (i) Expression of mutant mSAT-I in Escherichia coli

Expression of mutant mSAT-I in *Escherichia coli* was performed in a Cold Shock Expression System (manufactured by TaKaRa Bio Co., Ltd.).
Specifically, an *Escherichia coli* BL21 strain was transformed with Chaperon plasmid pG-Tf2 (manufactured by TaKaRa Bio Co., Ltd.) to create BL21-pG-Tf2 and then competent cells of the *Escherichia coli* BL21 strain were prepared. Then, a (ΔTM) mutant mSAT-I gene in which a transmembrane region (TM) was deleted was introduced into SAT-I expression plasmids (Psu141 and Psu142) and the competent cell was transformed again with these plasmids to create BL21-pG-Tf2-pSU141 and BL21-pG-Tf2-pSU142. The cells were preincubated in 1 ml of an LB medium (tryptone, yeast extract, NaCl, pH 7.2) at 37°C and then the preincubated cells were added to the LB medium so that the preincubated cells are diluted such that optical density OD at 600 nm was 0.1. At the same time, tetracycline was added up to 5 ng/ml and the cells were cultured at 37°C until optical density OD at 600 nm was 0.53. Then, the culture temperature was changed to 15°C and culture was continued for additional 30 minutes. After that, IPTG (isopropyl β-D-thiogalactopyranoside/manufactured by SIGMA) was added to the medium up to 0.01 mmol/l and the cells were cultured at 15°C for 48 hours. After completion of the culture, cells were collected by centrifugation. The collected cells were suspended in PBS containing 1 ml of 1 mmol/l fluorinated 4-(2-aminoethyl)benzenesulfonyl hydrochloride (ABESF; protease inhibitor) and sonicated twice for 30 seconds, and then centrifuged at 20,000×g for 15 minutes to collect a supernatant.
As a control, expression of wild type ΔTM-mSAT-I was similarly performed.

### (ii) Purification of mutant mSAT-I expressed in Escherichia coli

Then, purification of mutant mSAT-I was performed. First, Ni-NTA superflow (manufactured by QIAGEN) was aliquoted in a 1.5-ml tube so that the amount of gel was 10 µl, and 100 µl of PBS containing 0.01% Triton X-100 was added thereto and the gel was allowed to be mixed well. Then, the gel was subjected to equilibration treatment and centrifuged to remove a supernatant. Further, the supernatant obtained in the section (i) mentioned above was aliquoted and gently mixed with the gel by tapping and then the mixture was allowed to react at 4°C for 2 hours in a rotary mixer. After the reaction, the reaction mixture was centrifuged to remove the supernatant and wash buffer (composition: 50 mM NaH₂PO₄ (pH 8.0), 300 mM NaCl, 20 mM imidazole, 0.01% Triton X-100) was added thereto. The resultant mixture was centrifuged again to remove a supernatant, and elute buffer (composition: 50 mM NaH₂PO₄ (pH 8.0), 300 mM NaCl, 125 mM imidazole) was added thereto. The resultant mixture was gently mixed by tapping and centrifuged again to collect the supernatant. The collected supernatant was used as an Ni-affinity-purified sample.
Similarly, wild type mSAT-I was purified.
Further, imidazole in the elute buffer mentioned above was dialyzed and substituted by 20 mM Tris hydrochloride (pH 7.2) and 150 mM NaCl and used as an enzyme source as described in the section (iii) below.

### (iii) Assay of activity of mutant mSAT-I

After a solvent (chloroform:methanol = 1:1) of LacCer solution was dried 4×Reaction mixture was added four times and LacCer was suspended by sonication. Further, only 50 µl of 3 mM CMP-sialic acid, 100 µl of an enzyme source, and buffer (composition: 20 mM Tris hydrochloride, 150 mM NaCl, pH 7.2) were added and the resultant was allowed to react at 37°C for 2 hours. Ganglioside GM3 was purified from the reaction solution by means of Sep-Pak Plus18 and then was applied to an HPTLC plate. At the same time, a standard substance of GM3 (0.25 or 0.05 µg) was added. As a developing solvent, chloroform/methanol/0.2% CaCl₂ (55/45/10) was used to separate lipids. All the lipids were thermally transferred from the HPTLC plate to a PVDF membrane. The obtained PVDF membrane was dried and then blocked with blocking buffer to allow a primary antibody (M2590 which is an IgM monoclonal antibody specific to GM3 was used) to react with the membrane in the blocking buffer. After the PVDF membrane was well washed with TBS-T buffer, the PVDF membrane was allowed to react with a secondary antibody (anti-murine IgM antibody labeled with HRP) in the blocking buffer. The PVDF membrane was well washed with TBS-T buffer, and then the PVDF membrane was allowed to develop chemiluminescences with an ECL kit (manufactured by Amersham Biosciences) and GM3 was detected with an X-ray film.
Similarly, wild type mSAT-I that was expressed in *Escherichia coli* was also assayed for its activity.
From the results (Fig. 21), 0.05 µg or less of GM3 was detected in the case of the wild type mSAT-I, and about 0.5 µg of GM3 was detected in the case of the mutant SAT-I. Those results suggested that the mutant mSAT-I had an activity 10 folds or more as compared with that of the wild type SAT-I.

These results indicated that the gene of the mutant SAT-I which was not modified with sugar chains was expressed in *Escherichia coli* and had an activity equivalent to that of the wild type. The mutant protein of the present invention is a protein which has an activity without sugar chain modification, so the mutant protein can be mass-produced by using microbial cells such as *Escherichia coli,* and thus is useful.

The protein of the present invention is an N-linked glycoprotein in which a sugar chain that participates in expression of various functions, which retains the physiological activity of the glycoprotein without modification of a part of or the whole of the N-linked sugar chains. Hence a protein that has a function equivalent to that of the N-linked glycoprotein before the mutation is introduced can be produced conveniently, rapidly, in large amounts, and at low cost and further the quality of the protein to be produced can be maintained at a constant level. Therefore, the protein of the present invention is extremely useful. By utilizing the polynucleotide of the present invention, production of the protein of the present invention can be performed more conveniently and more rapidly, so the polynucleotide of the present invention is extremely useful. Further, the method of the present invention is extremely useful because it allows physiological functions of glycoproteins to be expressed without sugar chain modification. As described above, the present invention is extremely useful as a tool for synthesis or the like of pharmaceuticals, reagents, and various substances.

### Industrial Applicability

The protein of the present invention has a function which is equivalent to that of the N-linked glycoprotein before the mutation is introduced, so it can be utilized as a tool for synthesis of pharmaceuticals, reagents, and various substances or the like. The polynucleotide of the present invention can be utilized as a tool for production of the protein of the present invention or the like.

## Claims

1. A mutant sialyltransferase protein comprising an amino acid sequence selected from SEQ ID NO: 6, SEQ ID NO: 8, SEQ ID NO: 10, SEQ ID NO: 12, SEQ ID NO: 14, SEQ ID NO: 16 and SEQ ID NO: 18.

2. A polynucleotide which encodes the protein according to claim 1.

3. A mutant sialyltransferase protein comprising an amino acid sequence of SEQ ID No: 4 wherein asparagine at position 180 is replaced with serine, asparagine at position 224 is replaced with lysine and threonine at position 336 is replace with glutamine.

## Patentansprüche

1. Ein mutiertes Sialyl-Transferase-Protein umfassend eine Aminosäuresequenz, die aus SEQ ID NO: 6, SEQ ID NO: 8, SEQ ID NO: 10, SEQ ID NO: 12, SEQ ID NO: 14, SEQ ID NO: 16 und SEQ ID NO: 18 ausgewählt ist.

2. Ein Polynukleotid, das das Protein nach Anspruch 1 kodiert.

3. Ein mutiertes Sialyl-Transferase-Protein umfassend eine Aminosäuresequenz nach SEQ ID No: 4, wobei Asparagin an Position 180 durch Serin ersetzt ist, Asparagin an Position 224 durch Lysin ersetzt ist und Threonin an Position 336 durch Glutamin ersetzt ist.

## Revendications

1. Protéine sialyl transférase mutante renfermant une séquence d'acides aminés choisie parmi SEQ ID NO: 6, SEQ ID NO: 8, SEQ ID NO: 10, SEQ ID NO: 12, SEQ ID NO: 14, SEQ ID NO: 16 et SEQ ID NO: 18.

2. Poly nucléotide codant pour la protéine conforme à la revendication 1.

3. Protéine sialyl transférase mutante renfermant une séquence d'acides aminés selon SEQ ID NO: 4, l'asparagine en position 180 étant remplacée par de la sérine, l'asparagine en position 224 étant remplacée par de la lysine et la thréonine en position 336 étant remplacée par de la glutamine.
